# EUROPEAN PATENT APPLICATION

(11) **EP 1 593 692 A1**
(43) Date of publication of application: **09.11.2005**
(21) Application number: 04731497.6
(22) Date of filing: 06.05.2004
(51) Int. Cl.: C08B 37/08, C07H 5/06

(54) **CHITIN OLIGOMER COMPOSITION AND/OR CHITOSAN OLIGOMER COMPOSITION, AND PROCESS FOR PRODUCING THE SAME**

(30) Priority: 07.05.2003 JP 2003128660; 07.05.2003 JP 2003128644; 07.05.2003 JP 2003128646; 13.06.2003 JP 2003168693
(71) Applicant: Research Institute for Production Development, Kyoto 606-0805 (JP); Stella Chemifa Corporation, Osaka-shi, Osaka 541-0047 (JP)
(72) Inventor: KITANO, Hisao, Sakai-shi, Osaka 590-0023 (JP); SHIMIZU, Hiroshi, Ibi-gun, Gifu 501-0552 (JP); NOZAKI, Masakatsu Dai2kumemansyon 4-7, Kyoto-shi, Kyoto 606-8103 (JP); TANIMOTO, Fumio Muromachisukaihaitsu 112, Kamigyo-ku Kyoto-shi, Kyoto 602-8256 (JP); KIKUYAMA, Hirohisa c/o Stella Chemifa, Izumiohtsu-shi, Osaka 595-0075 (JP); WAKI, Masahide c/o Stella Chemifa, Izumiohtsu-shi, Osaka 595-0075 (JP); ITO, Kanenori c/o Stella Chemifa, Sakai-shi, Osaka 590-0982 (JP); HASHIGUCHI, Shinji c/o Stella Chemifa, Izumiohtsu-shi, Osaka 595-0075 (JP)
(74) Representative: von Kreisler, Alek
(86) International application number: PCT/JP2004/006429
(87) International publication number: WO 2005/005485

(57) **Abstract**

This invention aims to develop a chitin oligomer composition and/or a chitosan oligomer composition wherein the content of an oligomer from a trimer to a decamer is 60% or more, and to develop a process for preparing the above-mentioned oligomer composition. The object of the invention can be achieved by mixing hydrofluoric acid or hydrohalogenated acid other than hydrofluoric acid and hydrofluoric acid with a chitin-based material and/or a chitosan-based material.

## Description

### FIELD OF THE INVENTION

The present invention relates to a chitin oligomer composition and/or a chitosan oligomer composition, and to processes for preparing a chitin oligomer composition and/or a chitosan oligomer composition from a chitin-based material and/or a chitosan-based material and purifying the same to bring an industrial advantage. Specifically, the present invention concerns with a composition containing a chitosan oligomer and/or a chitin oligomer, and processes for preparing and purifying the same. More specifically, this invention is directed to processes for efficiently preparing and purifying a composition containing a chitosan oligomer and/or a chitin oligomer in the range from a trimer to a decamer. To take a more suitable example, the invention is directed to processes for efficiently preparing and purifying a composition containing a chitosan oligomer and/or a chitin oligomer in the range from a tetramer to a decamer. In addition, this invention is directed to processes for preparing and purifying a composition containing a chitosan oligomer and/or a chitin oligomer without imposing a load on a purifying step. In the specification, the chitosan oligomer and/or the chitin oligomer includes salts thereof.

### BACKGROUND ART

The chitin is a component which forms an exoskeleton for covering the outer surface of Crustacea (e.g., crabs, lobsters and euphausians), insects and like mollusks. The chitin is contained in the carapace of a cuttlefish or the tendon of a crab, shrimp or the like. In the domain of plants, chitins exist in the cell walls of molds, yeasts, mushrooms, green algae and the like.

The chitin is a polysaccharide with N-acetyl-D-glucosamine bonded to β-(1,4) and is a biomass which is comparable to cellulose, i.e. a structural factor of the plant. The chitin, when deacetylated, is called a chitosan. Most of chitosans are synthesized from chitins. When the deacetylation degree is in a region wherein it is difficult to determine whether a deacetylated chitin remains a chitin or has been converted to a chitosan, the chitin may be called a partially deacetylated chitin.

A chitin-based material and/or a chitosan-based material generally has a high biological activity for organisms, especially animals. Owing to this property, the chitin-based material and/or the chitosan-based material is widely used, e.g., for functional vegetable fibers, additives for feeds, feed caking agents, additives for foods, antibacterial agents, antiseptic agents, wound-healing agents, arthropathy-treating agents, raw materials for health foods, raw materials for cosmetics, waste water treating agents, soil conditioners, natural agricultural chemical components, and so on. The chitin-based material and/or the chitosan-based material is also widely used for cationic coagulants, coating compositions and dyes and for applications in the textile industry, the paper manufacturing industry or other industries.

However, because of the low solubility in water which results from a high crystallinity, the applications of these materials were neither diversified nor deeply investigated.

Generally a chitin oligomer or a chitosan oligomer is a low polymer prepared by the decomposition of a chitin-based material or a chitosan-based material. They have been improved in solubility in water and actively researched on their extended use. Especially chitin oligomers and chitosan oligomers containing monomers and dimers at a high ratio have been researched mainly about their use for foods because of their excellent solubility in water.

Chitin oligomers and chitosan oligomers, i.e., low polymers of chitin-based and/or chitosan-based materials, are mostly mixtures of oligomers which are different from each other in polymerization degree. In recent years, researches have been made about the widened use of chitin oligomers and/or chitosan oligomers in specific polymerization degrees. The possibility of broadening the use of chitin oligomers and/or chitosan oligomers with a specific polymerization degree is reported, for example, in S. Suzuki et al, Chem. Pharm. Bull., vol.33 (2), pp. 886-888, 1985; Carbohydrate Research, vol.151, pp. 403-408, 1986; Microbiol. Immunol., vol.30 (8), pp. 777-787, 1986; Chem. Pharm. Bull., vol.36 (2), pp. 784-790, 1988; and Microbiol. Immunol., vol.33 (4), pp. 357-367, 1989.

The inventors of the present invention conducted extensive research about the solubility and decomposition properties of polymers in highly concentrated hydrohalogenated acid. For example, in Japanese Unexamined Patent Publication Hei 11-5866, the present inventors proposed the use of hydrofluoric acid as an excellent solvent for fractionally dissolving a linear polyester.

Like cellulose, the chitin is not easily dissolved in a solvent. Known solvents are relatively limited and include, for example, concentrated sulfuric acid, concentrated phosphoric acid, hydrofluoric anhydride, formic anhydride, borofluoric mono- or di-hydrate, and thermally saturated aqueous solution of lithium rhodanate. Unlike cellulose, the chitin is defective in that the reaction mixture becomes black although the chitin can be dissolved in these solvents. This phenomenon presumably occurs because the produced glucosamine is subjected to air oxidation. When cellulose is dissolved in these acids, the cellulose oligomer forms an ester with the foregoing acids. The same phenomenon is seen in the case of a chitin oligomer and/or a chitosan oligomer. For instance, when a chitin is reacted with hydrofluoric anhydride, the chitin oligomer composition and the chitosan oligomer composition are lowered in purity. Thus, labor is generally required in purification of the contemplated product. When a chitin-based material and/or a chitosan-based material is decomposed in an enzyme, the resulting product is likewise lowered in purity with a by-product, and is increased in the concentration of endotoxin, frequently necessitating labor in purification of the contemplated product. The enzyme reaction proceeds with low efficiency because of poor solubility of a chitin-based material and/or a chitosan-based material.

The chitin-based material and/or the chitosan-based material is smoothly dissolved and decomposed in hydrogen halide, and can be liquefied within a relatively short time. However, when a large amount of hydrogen halide is reacted with a chitin-based and/or a chitosan-based material by contact with each other at one time, a large quantity of by-products is produced, resulting in tendency to entail labor in purification of the contemplated product.

Generally a neutralization method is carried out in recovering the chitin oligomer and/or the chitosan oligomer from a solution containing hydrogen halide. However, the method is likely to use enlarged equipment for control of intensive heat in a neutralization reaction between hydrogen halide and a basic component. If nothing is done to control the heat of neutralization reaction, a side reaction may occur because of an elevated temperature of the solution, thereby leading to reduction in the yield of the contemplated chitin oligomer and/or chitosan oligomer. It may be possible to conduct a method wherein a poor solvent for the chitin oligomer and/or the chitosan oligomer is mixed with the solution so that an oligomer is recovered from the precipitate. However, this method requires a large amount of a solvent for the solution containing a chitin oligomer and/or a chitosan oligomer. Thus large-scale equipment is needed and increases the production costs in commercial manufacture.

In carrying out the neuralization method, a neutral salt is produced. To prevent the neutral salt from its incorporation into a chitin oligomer composition and /or a chitosan oligomer composition, it is necessary to conduct a purification step using, e.g., a desalination device which is expensive and entails high maintenance costs.

According to the inventors' knowledge, a low temperature state must be kept in a method wherein 95 to 100% hydrogen halide is mixed with a chitin-based material and/or a chitosan-based material, the oligomer may further decompose easily due to the elevation of the temperature during the production operation of a chitin oligomer composition and/or a chitosan oligomer composition. Thus it is difficult to produce a chitin oligomer and/or a chitosan oligomer of a specific polymerization degree with a high reproducibility.

For this reason, prior art remains unsatisfactory in efficiently producing a chitin oligomer and/or a chitosan oligomer of a polymerization degree, e.g., in the range of a trimer to a decamer.

Referring to the compositions, oligosaccharides commercially available at the market are chitin oligomers and chitosan oligomers containing a monomer and a dimer at a high ratio, or containing at a high ratio an oligomer of a polymerization degree from a decamer or higher. It is still difficult to provide a chitin oligomer and a chitosan oligomer containing at a high ratio an oligosaccharide of a polymerization degree in the range from a tetramer to a decamer.

This is because it is difficult to control the decomposition of high molecular weight chitin and chitosan. A chitin oligomer and a chitosan oligomer are produced by the decomposition of high molecular weight chitin and chitosan. The production methods are roughly divided into a method utilizing an enzyme reaction, a method utilizing an acid hydrolysis reaction, and a method comprising a combination thereof. Numerous proposals have been offered about these methods. The decomposition reaction can not be controlled with ease, so that it is difficult to produce a chitin oligomer and/or a chitosan oligomer in the range of a tetramer to a decamer in a high yield.

It is a matter of course that industrially valuable are a chitin oligomer and a chitosan oligomer containing a monomer and a dimer at a high ratio, and a chitin oligomer and a chitosan oligomer containing at a high ratio an oligomer at a higher molecular weight side than a decamer. It can be merely said that chitin oligomers and chitosan oligomers containing at a high ratio oligosaccharides from a tetramer to a decamer are insufficient in application to purposes which exhibit a high function.

Further, a chitin and a chitosan contain heavy metals as impurities irrespective of their molecular weight. Thus there is room to improve the safety.

### DISCLOSURE OF THE INVENTION

The present inventors conducted extensive investigations to resolve the foregoing problems and found that the problems can be overcome by a fluorine-containing chitin oligomer and/or chitosan oligomer and by mixing a chitin-based material and/or a chitosan-based material with hydrofluoric acid in a specific concentration. Based on this finding, the invention was completed.

The invention provides a chitin oligomer composition and/or a chitosan oligomer composition characterized by containing 0.001 to 1000 ppm of fluorine atom and a process for preparing a chitin oligomer composition and/or a chitosan oligomer composition, the process being characterized by adding hydrofluoric acid in a concentration of 20 wt% to 95 wt.% to a chitin-based material and/or a chitosan-based material.

The invention further provides a process for preparing a chitin oligomer composition and/or a chitosan oligomer composition, the process being characterized in that at least one of (A) hydrofluoric acid and (B) hydrohalogenated acid other than hydrofluoric acid is added to a chitin-based material and/or a chitosan-based material; that the concentration of hydrofluoric acid is 1 to 75 wt% based on the total amount of hydrohalogenated acid other than hydrofluoric acid and hydrofluoric acid; and that the concentration of hydrohalogenated acid other than hydrofluoric acid is 1 to 35 wt.% based on the total amount of hydrohalogenated acid other than hydrofluoric acid and hydrofluoric acid, and a process for preparing a chitin oligomer composition and/or a chitosan oligomer composition, the process being characterized in that hydrohalogenated acid (except hydrofluoric acid) is added to a chitin oligomer and/or a chitosan oligomer which is one of a chitin-based material and a chitosan-based material.

First of all, the compositions of the invention will be described below.

In the invention, a chitin oligomer and/or a chitosan oligomer essentially contains 0.001 to 1000 ppm of fluorine atom. The amount of fluorine atom is suitably 0.001 to 100 ppm, more suitably 0.001 to 10 ppm, most suitably 0.01 to 10 ppm. When the amount of fluorine atom is less than 0.001 ppm, there is likely to arise a tendency to unsatisfactorily reduce the content of heavy metal, or a tendency to increase the production cost by taking a procedure for removal of heavy metal. On the other hand, when the oligomer contains more than 1000 ppm of fluorine atom, the fluorine-containing compound is likely to act as an impurity, and is undesirable from the viewpoint of safety.

In the invention, a chitin oligomer and/or a chitosan oligomer preferably contains 0.001 to 1000 ppm of calcium atom. The amount of calcium atom is suitably 0.001 to 100 ppm, more suitably 0.001 to 10 ppm, most suitably 0.01 to 10 ppm. When the amount of calcium atom is less than 0.001 ppm, there is likely to arise a tendency to unsatisfactorily reduce the content of heavy metal, or a tendency to increase the production cost owing to a need for a procedure for removal of heavy metal. On the other hand, when the oligomer contains more than 1000 ppm of calcium atom, the calcium atom (mainly calcium fluoride) is apt to act as an impurity, and is undesirable from the viewpoint of safety.

In the invention, a chitin oligomer and/or a chitosan oligomer contains suitably 60% or more of oligomer in the range of a trimer to a decamer, more suitably 70% or more of oligomer in the range of a trimer to a decamer, most suitably 80% or more of oligomer. When a chitin oligomer and/or a chitosan oligomer contains less than 60% of oligomer in the range of a trimer to a decamer, a more amount of the oligomer is dissolved in water, the oligomer tends to be more undistinguishable from the conventional chitin oligomer and/or chitosan oligomer containing a monomer or a dimer in a high concentration, or a less amount of the oligomer is dissolved in water, and the oligomer tends to be more undistinguishable from the conventional chitin oligomer and/or chitosan oligomer containing an oligomer from a decamer to a higher molecular weight side in a high concentration.

In the invention, a chitin oligomer and/or a chitosan oligomer contains suitably 40% or more of an oligomer in the range of a tetramer to a decamer, more suitably 50% or more of an oligomer in the range of a tetramer to a decamer, most suitably 60% or more of an oligomer in the range of a tetramer to a decamer. When a chitin oligomer and/or a chitosan oligomer contains less than 40% of oligomer in the range of a tetramer to a decamer, a more proportion of the oligomer is dissolved in water and the oligomer tends to become undistinguishable from the conventional chitin oligomer and/or chitosan oligomer containing a monomer or a dimer in a high concentration, or a less proportion of oligomer is dissolved in water, and the oligomer tends to become undistinguishable from the conventional chitin oligomer and/or chitosan oligomer containing an oligomer in the range of a decamer to a higher molecular weight side in a high concentration.

In the invention, the amount of the chitin oligomer and/or the chitosan oligomer dissolved in 100 g of water at 20°C is suitably 2 to 20 g. When the amount of oligomer dissolved in 100 g of water at 20°C is less than 2 g, the oligomer tends to become undistinguishable from the conventional chitin oligomer and/or chitosan oligomer containing an oligomer in the range of a decamer to a higher molecular weight side in a high concentration. When the amount of oligomer dissolved in 100 g of water at 20°C is more than 20 g, the oligomer tends to become undistinguishable from the conventional chitin oligomer and/or chitosan oligomer containing an oligomer from a monomer or a dimer in a high concentration.

In the invention, it is desirable that the chitin oligomer and/or the chitosan oligomer contains endotoxin in a concentration of 100 EU/ml or less.

In order to prevent the exposure of human body to an unexpected danger, the concentration of endotoxin is suitably less than 10 EU/ml, more suitably less than 1 EU/ml, most suitably in an undetected range.

More comments are added below.

The chitin oligomer composition and/or the chitosan oligomer composition prepared in the invention may contain another component (third component) such as lactose, saccharose, starch, glucose, crystalline cellulose, alginic acid, polylactic acid and the like. Further the composition may contain polyvinyl alcohol, ethylene-vinyl alcohol copolymer (ethylene content 15% or lower) and the like as the third component when used for other purposes than for feeds, foods and the like to be orally taken directly into the human body or animal body. The chitin oligomer and/or the chitosan oligomer may be used in mixture. From the viewpoint of industrial use, a less distribution of deacetylation degree is suitable. The content of impurities is preferably 0.1% or less, more preferably 0.01% or less, most preferably 0.001% or less.

The chitin oligomer composition and/or the chitosan oligomer composition according to the invention may be provided in the form of either a solid (powder or mass) or a liquid (solution or dispersion slurry). The chemical formulation of the composition may be a salt of single substance of chitin oligomer and/or chitosan oligomer or a salt of a mixture thereof. Salts of inorganic acids include, e.g., a salt of hydrofluoric acid. In view of corrosiveness and toxicity due to hydrofluoric ion, the composition may be converted into a safe anion (such as hydrochloride, acetate, lactate or salicylate), e.g., by a double decomposition method to facilitate the use.

It is suitable that the chitin oligomer composition and/or the chitosan oligomer composition of the invention is in the form of a chitin oligomer composition in that heavy metals can be highly removed.

Processes for preparing the composition will be described next.

The chitin-based material and/or the chitosan-based material for use in the invention is at least one species selected from a chitin which is a polysaccharide with N-acetyl-D-glucosamine bonded to β-(1 →4), a deacetylated chitin (partially deacetylatd chitin) and a chitosan, and includes a chitin oligomer and/or a chitosan oligomer. The chitin-based material and/or the chitosan-based material according to the invention includes chitins collected from Crustacea, insects, mollusks or microorganisms, and those, if necessary, as purified and recovered.

Crustacea, insects, mollusks or microorganisms can be directly used in the range wherein the effect of the invention is not impaired. Also available are chitins and chitosans deacetylated or partially deacetylated by treating them with sodium hydroxide or the like. Among them, preferable are those which are collected from Crustacea, especially lobsters and purified or recovered since these raw materials are inexpensive and can be stably obtained.

There is no limitation on the deacetylation degree of the chitin-based material and/or chitosan-based material in the invention. Since the concentration of remaining fluorine component in the chitin oligomer composition and/or the chitosan oligomer composition can be easily decreased, the deacetylation degree derived from the chitin-based material and/or the chitosan-based material is preferably 0.25 or less, more preferably 0.10 or less. It is desirable to use the chitin-based material rather than the chitosan-based material as the chitin-based material and/or the chitosan-based material because the use of a chitin-based material tends to reduce labor entailed in removal of undesired remaining fluorine component from the chitin oligomer composition and/or the chitosan oligomer composition.

The chitin-based material and/or the chitosan-based material in the invention is not limited in the shape but is preferably in the form of crushed particles. The particles to be used are preferably those which can pass through sieves of preferably 4-mesh, more preferably 16-mesh, most preferably 50-mesh.

In one of processes for preparing a chitin oligomer composition and/or a chitosan oligomer composition as the contemplated product of the invention, a chitin-based material and/or a chitosan-based material is reacted with hydrofluoric acid. It is important that the concentration of hydrofluoric acid to be used is 20 to 95 wt%. Preferred concentration is 20 to 60 wt%. When hydrofluoric acid is used in a concentration of 75 to 95 wt%, it is easy to increase the composition ratio of oligosaccharide at a low molecular weight side in the range of a trimer to a decamer. In the case of 20 to 75 wt%, the composition ratio of oligosaccharide at a high molecular weight side in the range of a trimer to a decamer can be easily increased. When the concentration of hydrofluoric acid to be used is lower, there is a tendency to produce at a high selectivity a chitin oligomer composition and/or a chitosan oligomer composition in the range of a trimer to a decamer. However, it is necessary to mix and react hydrofluoric acid with a chitin-based material and/or a chitosan-based material at a high temperature range or for a longer reaction time, and the composition is apt to be produced in a low yield. When the concentration of hydrofluoric acid is high, this brings a merit that a chitin oligomer composition and/or a chitosan oligomer composition is produced in a short time. But in this case, the process necessitates a reaction control such as a temperature control at a high level. Even if the process is conducted under a high-level control, this is likely to entail a high risk of producing a chitin oligomer composition and/or a chitosan oligomer composition from a trimer to a decamer at a low selective production efficiency and there is a tendency to increase a risk of involving an irregularity in distribution of polymerization degree and an increase of impurities, i.e. low reproducibility, concerning the oligomer obtained at every production lot.

When hydrofluoric acid is used in a concentration of less than 20 wt%, the process may be carried out at a high temperature and under a high pressure. On the other hand, when hydrofluoric acid is used in a concentration exceeding 95 wt%, this tends to produce an oligomer in a low yield and to give an oligomer in the range of a tetramer to decamer at a lower selective productivity. Further it becomes difficult to control the irregularity of formulation of a chitin oligomer composition and/or a chitosan oligomer composition obtained at every production lot. Moreover, in this case, the production cost is increased from the viewpoint of waste acid disposal.

In the invention, there is no restriction on the proportions of hydrofluoric acid and a chitin-based material and/or a chitosan-based material to be used in the reaction with each other in the invention. It is preferred to use hydrofluoric acid in an amount of preferably 10 to 100000 wt parts, more preferably 10 to 10000 wt parts, most preferably 100 to 1000 wt parts, per 100 wt parts of the chitin-based material and/or the chitosan-based material. When hydrofluoric acid is used in an amount of less than 10 wt parts per 100 wt parts of the chitin-based material and/or the chitosan-based material, the chitin-based material and/or the chitosan-based material is unlikely to uniformly react. When hydrofluoric acid is used in an amount of more than 100000 wt parts, the production cost may be increased because of cost for disposing of the used hydrofluoric acid. When hydrofluoric acid is subjected to neutralization, the temperature is exceedingly elevated due to neutralization heat. If cooling equipment is installed for overcoming the objection, various problems would arise such as degradation of properties resulting from secondary hydrolytic reaction, unexpected production of lower molecular weight product, change of deacetylation degree, production of a colored product by decomposition of the oligomer, and so on.

In the invention, there is no limitation on the method for reacting hydrofluoric acid with a chitin-based material and/or a chitosan-based material. The method will do if the chitin-based material and/or the chitosan-based material can be satisfactorily contacted with hydrofluoric acid. For example, the chitin-based material and/or the chitosan-based material may be incorporated into or added dropwise to hydrofluoric acid. Any suitable means for stirring and mixing can be effectively employed. It is preferred, however, that the chitin-based material and/or the chitosan-based material is contacted (hereinafter referred to as merely "mixed") with hydrofluoric acid uniformly without a time lag, in that a chitin oligomer composition and/or a chitosan oligomer composition can be efficiently produced. For example, a method wherein a chitin and/or a chitosan is added to hydrofluoric acid with stirring at one time is suitably selected.

When a chitin-based material and/or a chitosan-based material is mixed and reacted with hydrofluoric acid, a batch-wise method or a continuous method can be selected without question.

No limitation is imposed in the invention on the temperature for mixing and reacting a chitin-based material and/or a chitosan-based material with hydrofluoric acid. For example, it is between -20°C and 150°C, preferably between 25°C and 150°C, more preferably between 30°C to 120°C, most preferably between 35°C to 80°C. When the temperature is lower, the reaction slowly proceeds so that the control of reaction is easy. However, if the reaction proceeds too slowly, it is difficult to continue the hydrolysis reaction of a chitin-based material and/or a chitosan-based material until the intended polymerization degree is attained. When the temperature is higher, the reaction is too intensive, so that a an irregular distribution of polymerization degree tends to occur concerning the oligomer obtained at every production lot. In this case, glucosamine is produced in a large amount, and a chitin oligomer composition and/or a chitosan oligomer composition may be produced in a lower yield.

In the invention, it is preferable that a chitin-based material and/or a chitosan-based material is mixed and reacted with hydrofluoric acid under a circumstance which is shielded from oxygen or light. More preferably the circumstance is shielded from oxygen and light. The mixture tends to change the color to from thick brown to thin brown under a circumstance where light and oxygen exist.

In the invention, the deacetylation degree after contact with hydrofluoric acid tends to increase compared with the deacetylation degree of the chitin-based material and/or the chitosan-based material but generally scarcely changes. The deacetylation degree may be adjusted in a desired procedure after contact with hydrofluoric acid to obtain a chitin oligomer composition and/or a chitosan oligomer composition differing in deacetylation degree from that of a chitin-based material and/or a chitosan-based material. Specifically, when a chitin-based material is used, the conventional deacetylation reaction is carried out with a solution of sodium hydroxide, a solution of potassium hydroxide, a solution of calcium hydroxide or the like, whereby the chitin oligomer obtained by hydrolysis with hydrofluoric acid is deacetylated and can be converted to a chitosan oligomer. When using a chitosan-based material, the chitosan oligomer produced by hydrolysis with hydrofluoric acid can be converted, of course, to a chitin oligomer by the conventional acylation reaction.

The fluorine component derived from hydrofluoric acid is preferably removed from a solution containing a mixture of chitin-based material and/or chitosan-based material and hydrofluoric acid in a specified time after mixing them.

The method of removing the fluorine component derived from hydrofluoric acid is not limited and can be selected from conventional techniques such as a neutralization method, a reprecipitation method and the like. Especially the neutralization method is preferably selected.

In removing the fluorine component derived from hydrofluoric acid by the netralization method, generally a neutral system and/or a compound to be added to the neutral system is cooled to suppress the increase of temperature attributable to neutralization heat.

An HF concentration is adjusted preferably to 25% or less, more preferably to 20% or less in diluting the mixture with water or a hydrophilic organic solvent before neutralization, whereby the yield is increased and the content of organic impurities is reduced.

Examples of compounds useful in the neutralization method are sodium hydroxide, potassium hydroxide, calcium hydroxide and like hydroxides, calcium carbonate, sodium hydrogen carbonate and like carbonates, and ammonia and like basic substances. It is suitable to use calcium hydroxide, calcium carbonate and like basic calcium salts in that the reaction product obtained by the neutralization reaction can be easily removed and that the concentration of remaining fluorine component can be easily reduced.

The amount of the compound to be used is not limited. It is preferable to determine the amount of the compound so as to bring the liquidity of the solution to neutrality or basicity with a pH of 7 or higher, more preferably to basicity with a pH of 8 or higher since the decrease in the concentration of fluorine component in the solution is realized in a higher degree.

The solution adjusted to neutrality or basicity, preferably to basicity is preferably brought to neutrality or acidity with a pH of 6 or less after removal of undissolved components to further reduce the concentration of fluorine component, or is more preferably brought to acidity with a pH of 6 or less. In this case, the concentration of fluorine component in the solution is more effectively lowered by adding calcium chloride, calcium nitrate, calcium sulfate, calcium carbonate and like calcium salts for coexistence with the acidic substance.

The salt produced by neutralization is different depending on the basic substance used. Examples of such salts are calcium fluoride, sodium fluoride and so on. Generally the salt produced by neutralization is eliminated by a desalting device such as an electrodialyser, and the device can be used in the invention. The concentration of residual neutral salt can be lowered without use of a special desalting device. This is one of the features of the invention.

The fluoride has a low solubility and calcium fluoride is especially low in solubility so that the concentration of neutralization salt remaining in the solution can be reduced by simple means such as filtration without use of a special device such as an electrodialyser in the invention.

The solution of a chitin oligomer and/or a chitosan oligomer prepared by mixing a chitin-based material and/or a chitosan-based material with hydrofluoric acid can be used as such according to the purpose or can be used as a powder after being dried by concentration. The composition can be adjusted or can be highly purified using an RO membrane, ultrafiltration membrane or like membrane fractionation, HPLC or like chromatographic fractionation.

In a step after removing the fluorine component derived from hydrofluoric acid from the solution containing hydrofluoric acid, the solution containing a chitin oligomer and/or a chitosan oligomer is contacted with at least one species selected from activated carbon, alumina, silica gel, alkyl-containing silica gel, amino-containing silica gel, hydroxyl-containing silica gel, and cyano-containing silica gel, whereby the purpose can be achieved, i.e. the purpose of reducing the distribution of polymerization degree of the chitin oligomer and/or chitosan oligomer contained in the chitin oligomer composition and/or chitosan oligomer composition, or the purpose of removing the impurities from the chitin oligomer composition and/or chitosan oligomer composition.

A solution containing a chitin oligomer and/or a chitosan oligomer is passed through a container packed with at least one species selected from activated carbon, alumina, silica gel, alkyl-containing silica gel, amino-containing silica gel, hydroxyl-containing silica gel and cyano-containing silica gel, whereby the chitin oligomer and/or chitosan oligomer and impurities are adsorbed on the filler. Then an elute is passed therethrough, whereby the impurities are removed from the chitin oligomer and/or the chitosan oligomer, and the oligomer can be fractionally recovered according to the polymerization degree.

The elute may not be at room temperature but may be warm water. When it is warm water, the dimer can be effectively removed and an oligosaccharide of the intended composition is likely to be easily obtained.

The elute can be suitably selected according to the purpose depending on the kind of the filler.

The chitin oligomer composition and/or a chitosan oligomer composition obtained according to the invention can be purified also by recrystallization.

The solvent to be used in purification is not limited. Suitable examples of the solvent are water; alcohols such as methanol, ethanol, 1-propanol, 2-propanol and 1-butanol; ketones such as acetone and methyl ethyl ketone; and carboxylic acids such as acetic acid and butyric acid.

A third component conventionally known may be suitably added in the range which does not impair the effect of the invention. Examples of the third component are organic solvents, acids, alkali, antifoamers and heat stabilizers.

In the invention, a mixture of a chitin-based material and/or a chitosan-based material and hydrofluoric acid is mostly liquid. An organic solvent can be properly added to control the reaction activity and the solubility. Examples of the organic solvent are alcohols such as methanol, ethanol, 1-propanol, 2-propanol and 1-butanol; ketones such as acetone and methyl ethyl ketone; carboxylic acids such as acetic acid and butyric acid.

Among these organic solvents, a hydrophilic organic solvent is suitably used. For example, after a chitin-based material and/or a chitosan-based material is mixed with hydrofluoric acid for a specified time period, and in a stage before removal of fluorine component derived from hydrofluoric acid, a hydrophilic organic solvent is added in an amount of 1 to 400 wt parts per 100 wt parts of hydrofluoric acid, whereby a chitin oligomer composition and/or a chitosan oligomer composition with a polymerization degree of about 10 to about 20 is precipitated from the solution so that it can be recovered and removed by filtration or like means.

In the invention, the mixture of a chitin-based material and/or a chitosan-based material with hydrofluoric acid may contain a surfactant.

The surfactant effectively increases an affinity in an initial stage of mixing the chitin-based material and/or the chitosan-based material with hydrofluoric acid and markedly eliminates the possibility of low reproducibility of quality concerning the obtained chitin oligomer and/or chitosan oligomer. Since the wetting of chitin-based material and/or chitosan-based material is enhanced, the amount of hydrofluoric acid can be decreased relative to the chitin-based material and/or the chitosan-based material, and thus the remaining amount of the fluorine component derived from hydrofluoric acid can be reduced.

The amount of the surfactant to be added is not limited. The possibility of lower reproducibility of quality concerning the obtained product is significantly lowered when the surfactant is incorporated in an amount of 0.0001 to 1 wt% into the solution containing the chitin-based material and/or chitosan-based material and hydrofluoric acid. When the concentration of the surfactant is less than 0.0001 wt%, the wetting of a chitin-based material and/or a chitosan-based material is enhanced in a less degree. In the case of a concentration of higher than 1 wt%, it may become necessary to introduce equipment for exclusive use in removal of the surfactant.

The type of the surfactant to be used is not limited. Useful surfactants include conventional surfactants, e.g., anionic surfactants such as a salt of alkylsulfuric acid ester, a salt of alkylbenzenesulfonic acid and a salt of alkylphosphoric acid, cationic surfactants such as alkylamine salt and tetraammonium salt, nonionic surfactants such as polyoxyalkylene alkyl ether and glycerin fatty acid ester. Among them, surfactants free of metal elements are preferred.

A chitosan portion of the chitin oligomer and/or the chitosan oligomer produced by mixing a chitin-based material and/or a chitosan-based material with hydrofluoric acid is in the form of hydrohalogenated acid salt used. The chitin oligomer composition and/or the chitosan oligomer composition thus obtained is converted to the contemplated inorganic acid salt (e.g., hydrochloride) or organic acid salt (e.g., acetate, lactate or salicylate) by the conventional method such as double decomposition method and is put to practical use.

In mixing a chitin-based material and/or a chitosan-based material with hydrofluoric acid in the invention, hydrofluoric acid and hydrohalogenated acid other than hydrofluoric acid can be used in combination. This mode will be described below.

According to the invention, there are mixed together a chitin-based material and/or a chitosan-based material, hydrohalogenated acid other than hydrofluoric acid and hydrofluoric acid. In this procedure, the concentration of hydrofluoric acid is 1 to 75 wt.%, preferably 20 to 75 wt.%, based on the total amount of hydrohalogenated acid other than hydrofluoric acid and hydrofluoric acid. The concentration of hydrohalogenated acid other than hydrofluoric acid is 1 to 35 wt.% based on the total amount of hydrohalogenated acid other than hydrofluoric acid and hydrofluoric acid.

It is desirable to adjust the concentration of hydrofluoric acid to be used to 1 wt%, preferably 20 to 75 wt%, more preferably 20 to 60 wt%, based on the total amount of hydrohalogenated acid other than hydrofluoric acid and hydrofluoric acid. When the concentration of hydrofluoric acid is less than the range based on the total amount of hydrohalogenated acid other than hydrofluoric acid and hydrofluoric acid, a chitin oligomer composition and/or a chitosan oligomer composition from a trimer to a decamer is apt to be produced at a high selectivity. But this necessitates mixing and reaction of a chitin-based material and/or a chitosan-based material with hydrofluoric acid at a high temperature or for a longer reaction time. On the other hand, when the concentration of hydrofluoric acid is higher than the range based on the total amount of hydrohalogenated acid other than hydrofluoric acid and hydrofluoric acid, this gives a merit that a chitin oligomer composition and/or a chitosan oligomer composition can be produced in a short time. However, it is necessary to carry out the reaction control such as temperature control at a high level. Even if the production operation is conducted under a high-level control, there is a tendency to increase a risk of entailing an irregular distribution of polymerization degree and increased impurities leading to low reproducibility of quality concerning the oligomer obtained at every production lot.

When the concentration of hydrofluoric acid is less than 1 wt% based on the total amount of hydrohalogenated acid other than hydrofluoric acid and hydrofluoric acid, the operation is to be carried out at a high temperature and under a high pressure. On the other hand, when the concentration of hydrofluoric acid exceeds 70 wt% based on the total amount of hydrohalogenated acid other than hydrofluoric acid and hydrofluoric acid, the yield for the oligomer and especially the selective productivity of a tetramer to decamer tend to decrease, and it is likely to be difficult to suppress the deterioration of reproducibility of quality concerning the chitin oligomer composition and/or the chitosan oligomer composition produced at every production lot. In this case, the production cost is increased from the viewpoint of disposal of waste acid.

The hydrohalogenated acid other than hydrofluoric acid in the invention refers to at least one of hydrochloric acid, hydrobromic acid and hydriodic acid. It is desirable to adjust the concentration of hydrohalogenated acid other than hydrofluoric acid to preferably 1 to 35 wt%, more preferably 5 to 30 wt%, most preferably 10 to 30 wt%, based on the total amount of hydrohalogenated acid other than hydrofluoric acid and hydrofluoric acid. When the concentration of hydrohalogenated acid other than hydrofluoric acid is low relative to the total amount of hydrohalogenated acid other than hydrofluoric acid and hydrofluoric acid, a chitin oligomer composition and/or a chitosan oligomer composition from a trimer to a decamer tends to be produced at a high selectivity, although depending on the concentration of hydrofluoric acid. It is necessary to mix and react a chitin-based material and/or a chitosan-based material with hydrofluoric acid in a high temperature region or for a longer reaction time. On the other hand, when the concenration of hydrohalogenated acid other than hydrofluoric acid is high relative to the total amount of hydrohalogenated acid other than hydrofluoric acid and hydrofluoric acid, this gives a merit that a chitin oligomer composition and/or a chitosan oligomer composition can be produced in a short time. However, it is necessary to carry out the reaction control such as temperature control at a high level. Even if the production operation is conducted under a high-level control, there is increased a risk of giving rise to irregular distribution of polymerization degree and increased impurities, i.e. low reproducibility of quality concerning the oligomer obtained at every production lot.

When the concentration of hydrohalgenated acid other than hydrofluoric acid is less than 1 wt% based on the total amount of hydrohalogenated acid other than hydrofluoric acid and hydrofluoric acid, the reaction is to be carried out at a high temperature and under a high pressure although depending on the concentration of hydrofluoric acid. On the other hand, when the concentration of hydrohalogenated acid other than hydrofluoric acid exceeds 35 wt% based on the total amount of hydrohalogenated acid other than hydrofluoric acid and hydrofluoric acid, the yield for an oligomer and the selective productivity of a tetramer to a decamer tend to decrease, and it is difficult to suppress the deterioration of reproducibility of quality concerning the chitin oligomer composition and/or a chitosan oligomer composition obtained at every production lot. In this case, an increased production cost is incurred due to the disposal of waste acid.

The amount of hydrohalogenated acid other than hydrofluoric acid and hydrofluoric acid is preferably so controlled that the fluorine atom accounts for preferably 50% or more, more preferably 75% or more, of the total amount of halogen atom in the mixture of a chitin-based material and/or a chitosan-based material, hydrohalogenated acid other than hydrofluoric acid and hydrofluoric acid.

If the fluorine atom accounts for less than 50% in the total amount of halogen atom in the mixture of a chitin-based material and/or a chitosan-based material, hydrohalogenated acid other than hydrofluoric acid and hydrofluoric acid, a greater load is imposed on removal of the halogen component derived from hydrohalogenated acid other than hydrofluoric acid.

Due to coexistence of hydrohalogenated acid other than hydrofluoric acid with hydrofluoric acid, the reaction rate is likely to be high compared with the reaction of hydrohalogenated acid other than hydrofluoric acid and hydrofluoric acid in the same halogen concentration. In other words, the amount of halogen atom to be used can be reduced compared with single use of hydrohalogenated acid other than hydrofluoric acid and hydrofluoric acid.

In the invention, there is no limitation on the proportions of three ingredients, i.e. chitin-based material and/or chitosan-based material, hydrohalogenated acid other than hydrofluoric acid and hydrofluoric acid to be used in the reaction. The combined amount of hydrohalogenated acid other than hydrofluoric acid and hydrofluoric acid is preferably 10 to 100000 wt parts, more preferably 10 to 10000 wt parts, most preferably 100 to 1000 wt parts, per 100 wt parts of a chitin-based material and/or a chitosan-based material. When the combined amount of hydrohalogenated acid other than hydrofluoric acid and hydrofluoric acid is less than 10 wt parts, per 100 wt parts of a chitin-based material and/or a chitosan-based material, the chitin-based material and/or the chitosan-based material is unlikely to uniformly react. When the combined amount of hydrohalogenated acid other than hydrofluoric acid and hydrofluoric acid is more than 100000 wt parts, the production cost may be increased for disposal of the used hydrofluoric acid. In the case of subjecting hydrofluoric acid to neutralization treatment, the temperature is exceedingly elevated by neutralization heat. If cooling equipment is installed for overcoming this problem, various problems arise such as degradation of properties derived from secondary hydrolytic reaction, unexpected production of lower molecular weight product, change of deacetylation degree, production of colored product by decomposition of an oligomer, and the like.

In the invention, there is no restriction on the method for reacting a chitin-based material and/or a chitosan-based material, hydrohalogenated acid other than hydrofluoric acid and hydrofluoric acid. The reaction may be conducted by a suitable method such as adding dropwise hydrohalogenated acid other than hydrofluoric acid and hydrofluoric acid to the chitin-based material and/or the chitosan-based material, or incorporating the chitin-based material and/or the chitosan-based material into a mixture of hydrohalogenated acid other than hydrofluoric acid and hydrofluoric acid, or by effectively employing various means, e.g., stirring and mixing. It is desired to contact (hereinafter merely referred to by "mix") the chitin-based material and/or the chitosan-based material with hydrohalogenated acid other than hydrofluoric acid and hydrofluoric acid uniformly without a time lag to effectively produce a chitin oligomer composition and/or a chitosan oligomer composition. Suitably selectable are, for example, a method wherein a mixture of hydrohalogenated acid other than hydrofluoric acid and hydrofluoric acid is added at one time to the chitin-based material and/or the chitosan-based material with stirring, and a method wherein hydrohalogenated acid other than hydrofluoric acid and hydrofluoric acid are added at the same time to the chitin-based material and/or the chitosan-based material.

When a chitin-based material and/or a chitosan-based material is mixed and reacted with hydrohalogenated acid other than hydrofluoric acid and hydrofluoric acid, a batch-wise method or a continuous method can be selected without question.

Other reaction means and conditions are generally the same as when hydrofluoric acid is used.

The present invention includes the following.

The invention provides a method for mixing hydrohalogenated acid (other than hydrofluoric acid) with a chitin oligomer and/or a chitosan oligomer which is one of a chitin-based material and/or a chitosan-based material. The chitin oligomer and/or the chitosan oligomer is smoothly dissolved and decomposed by hydrohalogenated acid and can be liquefied in a relatively short time. However, if a large amount of hydrohalogenated acid is contacted with the chitin oligomer and/or the chitosan oligomer at one time for reaction, a large amount of by-products is so produced that labor is required for purification of the contemplated product.

The present inventors conducted extensive research to overcome the foregoing problem, and found that the problem can be overcome by adding hydrohalogenated acid (excepting hydrofluoric acid) to a chitin oligomer and/or a chitosan oligomer.

The invention also provides a process for preparing a chitin oligomer composition and/or a chitosan oligomer composition, the process being characterized by adding hydrohalogenated acid (excepting hydrofluoric acid) to a chitin oligomer and/or a chitosan oligomer.

The chitin oligomer and/or the chitosan oligomer refers to a chitin oligomer which is an oligosaccharide having N-acetyl-D-glucosamine bonded to β-(1→4), a partially deacetylated chitin oligomer and/or a chitosan oligomer.

In the invention, using hydrohalogenated acid (other than hydrofluoric acid), a chitin oligomer and/or a chitosan oligomer is prepared from, e.g. a chitin-based material and/or a chitosan-based material. More specifically, it is obtained, e.g., by acid hydrolysis of a chitin-based material and/or a chitosan-based material or by enzyme decomposition thereof.

A chitin oligomer and/or a chitosan oligomer to be used in the invention contains 70 wt% or more of an oligomer with a polymerization degree of 2 to 40. Used in the invention is preferably a chitin oligomer and/or a chitosan oligomer with a polymerization degree of 2 to 5 and/or a polymerization degree of 10 to 40, more preferably a chitin oligomer and/or a chitosan oligomer with a polymerization degree of 2 to 4 and/or a polymerization degree of 10 to 20.

There is no limitation on the deacetylation degree of an chitin oligomer and/or an chitosan oligomer to be used in the invention. Since the concentration of unwanted halide in the chitin oligomer composition and/or chitosan oligomer composition can be easily reduced, the deacetylation degree derived from the chitin oligomer and/or the chitosan oligomer is preferably 0.25 or less, more preferably 0.10 or less. When the chitin oligomer rather than the chitosan oligomer is used as the chitin oligomer and/or chitosan oligomer, less labor tends to be involved in removal of remaining unwanted halogen components from the chitin oligomer composition and/or the chitosan oligomer composition.

The chitin oligomer and/or chitosan oligomer according to the invention is not limited in the form but is preferably in the form of crushed particles. The particles to be used are those which can pass through sieves of preferably 4-mesh, more preferably 16-mesh, most preferably 50-mesh. Optionally the particles may be in the form of a solvent dissolved in a liquid.

In the process for preparing a chitin oligomer composition and/or a chitosan oligomer composition according to the invention, the chitin oligomer and/or the chitosan oligomer as the raw material is reacted with hydrohalogenated acid (other than hydrofluoric acid). The hydrohalogenated acid (other than hydrofluoric acid) is used in a concentration of preferably 1 to 70 wt%, more preferably 20 to 60 wt%. When the concentration of hydrohalogenated acid (other than hydrofluoric acid) is lower, there is a tendency of producing at a high selectivity a chitin oligomer composition and/or a chitosan oligomer composition in the range of a trimer to a decamer. However, it is necessary to mix and react hydrohalogenated acid with a chitin oligomer and/or a chitosan oligomer in a high temperature range or for a longer reaction time. When the concentration of hydrohalogenated acid (other than hydrofluoric acid) is higher, there is brought a merit that a chitin oligomer composition and/or a chitosan oligomer composition can be produced in a short time. However, in this case, the process necessitates a reaction control such as a temperature control at a high level. Even if the composition is produced under a high-level control, this operation is likely to entail a higher risk of giving rise to an irregular distribution of polymerization degree and increase of impurities, i.e., low reproducibility of quality concerning the oligomer obtained at every production lot. Another problem is a tendency for increasing the production cost from the viewpoint of waste acid disposal.

When hydrohalogenated acid (other than hydrofluoric acid) is added to a chitin oligomer and/or a chitosan oligmer in the invention, the proportions of the two components are not limited. It is preferred, however, to use hydrohalogenated acid (other than hydrofluoric acid) in an amount of preferably 10 to 100000 wt parts, more preferably 10 to 10000 wt parts, most preferably 100 to 1000 wt parts, per 100 wt parts of the chitin oligomer and/or the chitosan oligomer. When hydrohalogenated acid (other than hydrofluoric acid) is used in an amount of less than 10 wt parts per 100 wt parts of the chitin oligomer and/or chitosan oligomer, the chitin oligomer and/or chitosan oligomer is unlikely to uniformly react. When hydrohalogenated acid (other than hydrofluoric acid) is used in an amount of more than 100000 wt parts, the production cost may be increased because of cost for disposing of the used hydrohalogenated acid (other than hydrofluoric acid). When hydrohalogenated acid (other than hydrofluoric acid) is subjected to neutralization treatment, the temperature is exceedingly elevated due to neutralization heat. If cooling equipment is installed for overcoming the objection, various problems arise such as degradation of properties owing to secondary hydrolytic reaction, unexpected production of lower molecular weight product, change of deacetylation degree, production of colored product by decomposition of oligomer, and the like.

In the invention, there is no limitation on the method for adding (which may be hereinafter referred to simply as "mixing") hydrohalogenated acid (other than hydrofluoric acid) to a chitin oligomer and/or a chitosan oligomer in the invention. Hydrohalogenated acid (other than hydrofluoric acid) may be added dropwise to a chitin oligomer and/or a chitosan oligomer. Optionally a chitin oligomer and/or a chitosan oligomer may be incorporated to hydrohalogenated acid (other than hydrofluoric acid). However, it is preferred to mix a chitin oligomer and/or a chitosan oligomer with hydrohalogenated acid (other than hydrofluoric acid) uniformly without a time lag to produce a chitin oligomer composition and/or a chitosan oligomer composition efficiently. For example, a method wherein a chitin oligomer and/or a chitosan oligomer is added to hydrohalogenated acid (other than hydrofluoric acid) at one time with stirring is suitably selected.

When hydrohalogenated acid (other than hydrofluoric acid) is mixed and reacted with a chitin oligomer and/or a chitosan oligomer, a batch-wise method or a continuous method can be selected without question.

Other reaction means and conditions are the same as in use of a chitin-based material and/or a chitosan-based material other than a chitin oligomer and/or a chitosan oligomer as a rule.

In the invention, the deacetylation degree after contact with hydrohalogenated acid (other than hydrofluoric acid) tends to increase compared with the deacetylation degree of a chitin oligomer and/or a chitosan oligomer as the raw material but usually scarcely changes. The deacetylation degree may be adjusted in an optional procedure after contact with hydrohalogenated acid (other than hydrofluoric acid) to obtain a chitin oligomer composition and/or a chitosan oligomer composition differing in deacetylation degree from that of the chitin oligomer and/or the chitosan oligomer used as the raw material. Specifically, when using a chitin oligomer, the conventional deacetylation reaction is carried out with a solution of sodium hydroxide, a solution of potassium hydroxide, a solution of calcium hydroxide or the like, whereby the chitin oligomer obtained by hydrolysis with hydrohalogenated acid (other than hydrofluoric acid) can be deacetylated for conversion to a chitosan oligomer. When using a chitosan oligomer, the chitosan oligomer obtained by hydrolysis with hydrohalogenated acid (other than hydrofluoric acid) can be converted, of course, to a chitin oligomer by the conventional acylation reaction.

A chitosan portion of a chitin oligomer and/or a chitosan oligomer produced by mixing the oligomer with hydrohalogenated acid (other than hydrofluoric acid) assumes a form of hydrohalogenated acid (other than hydrofluoric acid) used. The chitin oligomer composition and/or the chitosan oligomer composition thus obtained can be converted to the contemplated salt of inorganic acid (e.g., hydrochloride) or salt of organic acid (e.g., acetate, lactate or salicylate) by the conventional method such as double decomposition method for practical use.

### BRIEF DESCRIPTION OF THE DRAWINGS

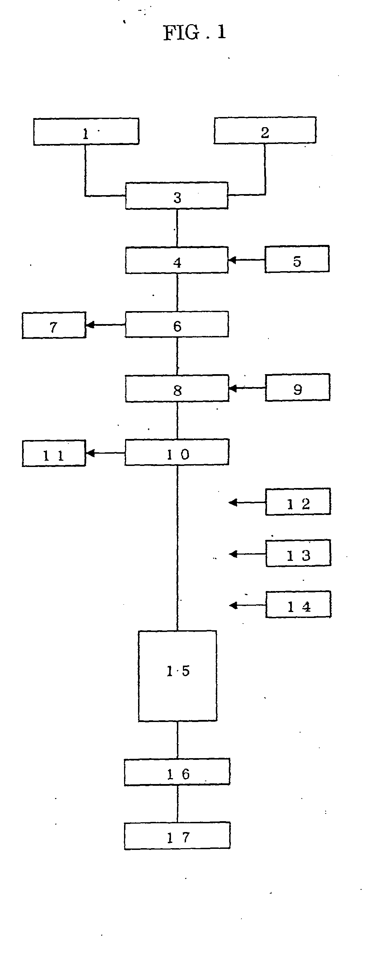

Fig.1 is a flow sheet showing the flow of steps in the process for preparing the composition of the invention.

The flow sheet of Fig.1 shows a specific flow of steps in the process for preparing a chitin oligomer composition and/or a chitosan oligomer composition according to the invention. The following reference numerals in Fig. 1 indicate:
1: Chitin-based material and/or chitosan-based material
2: Hydrofluoric acid and/or hydrohalogenated acid (other than hydrofluoric acid)
3: Reaction mixture
4: Mixture of the reaction mixture 3 and hydrophilic organic solvent
5: Hydrophilic organic solvent
6: Filtration
7: Partial removal (recovery) of chitin oligomer and/or chitosan oligomer
8: Filtrate
9: Basic calcium salt
10: Filtration
11: Removal of halide such as fluoride
12: pH adjuster
13: Filtration
14: Hydrophilic organic solvent
15: Column
16: Concentration to dryness
17: Contemplated product

The flow sheet of Fig.1 will be described in more detail. First of all, a chitin-based material and/or a chitosan-based material 1 is contacted for reaction with hydrofluoric acid and/or hydrohalogenated acid (other than hydrofluoric acid) 2 to give a reaction mixture 3. The temperature for the reaction is -20 to 150°C.

A hydrophilic organic solvent 5 is added to the reaction mixture 3 to give a mixture 4. The mixture 4 is subjected to filtration 6 for removal of part 7 of a chitin oligomer and/or a chitosan oligomer. A basic calcium salt 9 is added to the filtrate 8 until the filtrate 8 is made basic. The mixture is subjected to filtration 10 to remove a halide 11. A pH adjuster 12 is added, as required, to the filtrate. The pH adjuster 12 is used in view of use of silica or the like which is easily degradable in properties when made basic in a step involving a column 15.

A hydrophilic organic solvent 14 is added to a filtrate (obtained by filtration 13 when required), and then the mixture is passed through the column 15 wherein the contemplated product, i.e. a chitin oligomer composition and/or a chitosan oligomer composition is adsorbed in the column 15. The composition is removed by the flow of an elute.

Finally the contemplated product 17 is obtained by being dried by concentration 16.

### Field of the Invention

The invention relates to a chitin oligomer composition and/or a chitosan oligomer composition, and processes for preparing the same.

### BEST MODE FOR CARRYING OUT THE INVENTION

The invention will be described in more detail with reference to the following examples illustrating the invention.

### Example 1

Forty grams of 55% hydrofluoric acid was placed into a PFA container equipped with a stirrer. The air of the atmosphere was replaced by nitrogen gas. Then 10 g of chitin (product of Koyo Chemical Co., Ltd., Chitin TC-L) was placed into the container. The chitin and hydrofluoric acid became uniform in about 10 seconds. A reaction was carried out in this state at 40°C for 5 hours. Then 30 g of ethanol and 170 g of pure water were added, and undissolved components were removed by filtration.
The filtrate was neutralized with 55.0 g of calcium carbonate in a water bath. The solution had a pH of 7. After the solution was separated into solid and liquid by re-filtration, 0.1 N hydrochloric acid was added so as to attain a pH of 5. The solution was passed through a column 40 mm in diameter which was filled with 500 g of activated carbon (product of Wako Junyaku Co., Ltd.) useful for chromatography. Warm water (1000 g) was passed through the column and an aqueous solution (3000 g) of ethanol with a linear concentration gradient of ethanol concentration 5% to 30% was passed through the column.
The solution flowing out from the column was fractionally recovered and concentrated under reduced pressure, giving 0.3 g of chitin monomer, 0.7 g of chitin dimer, 1.5 g of chitin trimer, 2.0 g of chitin tetramer, 1.9 g of chitin pentamer, 1.3 g of chitin hexamer, 0.2 g of chitin heptamer or octamer and 0.1 g of chitin nonamer or decamer.

### Example 2

Forty grams of 50% hydrofluoric acid containing 10 ppm of Emazol L-10 (surfactant manufactured by Kao Corp.) was placed into a PFA container equipped with a stirrer. The air of the atmosphere was replaced by nitrogen gas. Then 10 g of chitin (Chimica Chitin F, product of Chimica Co., Ltd.) was placed into the container. The chitin and hydrofluoric acid became uniform in about 10 seconds. A reaction was carried out in this state at 40°C for 6 hours. Then 180 g of pure water was added, and undissolved components were removed by filtration. The filtrate was neutralized with 50.0 g of calcium carbonate in a water bath. The solution had a pH of 7. After the solution was separated into solid and liquid by re-filtration, 0.1 N hydrochloric acid was added so as to attain a pH of 5. The solution was passed through a column 40 mm in diameter which was filled with 500 g of activated carbon (product of Wako Junyaku Co., Ltd.) useful for chromatography. Warm water (2000 g) was passed through the column and an aqueous solution (5000 g) of ethanol with a linear concentration gradient of ethanol concentration 5% to 60% was passed through the column.
The solution flowing out from the column was fractionally recovered and concentrated under reduced pressure, giving 0.5 g of chitin dimer, 1.2 g of chitin trimer, 2.2 g of chitin tetramer, 2.2 g of chitin pentamer, 1.5 g of chitin hexamer, 0.4 g of chitin heptamer or octamer and 0.3 g of chitin nonamer or decamer.

### Example 3

Forty grams of 50% hydrofluoric acid containing 50 ppm of Leodol TW-L106 (surfactant manufactured by Kao Corp.) was placed into a PFA container equipped with a stirrer. The air of the atmosphere was replaced by nitrogen gas. Then 10 g of chitin (Chitin TC-L, product of Koyo Chemical Co., Ltd.) was placed into the container. The chitin and hydrofluoric acid became uniform in about 10 seconds. Thereafter a reaction was carried out in this state at 40°C for 3 hours. Then 30 g of methanol and 70 g of pure water were added, and undissolved components were removed by filtration. The filtrate was neutralized with 37.2 g of calcium hydroxide in an ice bath. The solution had a pH of 9. After the solution was separated into solid and liquid by re-filtration, 0.1 N hydrochloric acid was added so as to attain a pH of 5. The solution was passed through a column 40 mm in diameter which was filled with 500 g of activated carbon (product of Wako Junyaku Co., Ltd.) useful for chromatography. Pure water (1000 g) was passed through the column and an aqueous solution (3000 g) of methanol with a linear concentration gradient of methanol concentration 5% to 80% was passed through the column. The solution flowing out from the column was fractionally recovered and concentrated under reduced pressure, giving 2.0 g of chitin dimer, 1.5 g of chitin trimer, 1.0 g of chitin tetramer, 0.6 g of chitin pentamer, 0.4 g of chitin hexamer, 0.4 g of chitin heptamer or octamer and 0.2 g of chitin nonamer or decamer.

### Example 4

Forty grams of 45% hydrofluoric acid containing 10 ppm of Emazol L-10 (surfactant manufactured by Kao Corp.) was placed into a PFA container equipped with a stirrer. The air of the atmosphere was replaced by nitrogen gas. Then 10 g of chitin (Chimica Chitin F, product of Chimica Co., Ltd.) was placed into the container. The chitin and hydrofluoric acid became uniform in about 10 seconds. Thereafter a reaction was carried out in this state at 50°C for 4 hours. Then 80 g of pure water was added, and undissolved components were removed by filtration. The filtrate was neutralized with 33.4 of calcium hydroxide in an ice bath. The solution had a pH of 9. After the solution was separated into solid and liquid by re-filtration, 0.1 N hydrochloric acid was added so as to attain a pH of 5. The solution was passed through a column 40 mm in diameter which was filled with 500 g of activated carbon (product of Wako Junyaku Co., Ltd.) useful for chromatography. Pure water (1000 g) was passed through the column and an aqueous solution (3000 g) of methanol with a linear concentration gradient of methanol concentration 5% to 80% was passed through the column. The solution flowing out from the column was fractionally recovered and concentrated under reduced pressure, giving 2.5 g of chitin dimer, 1.8 g of chitin trimer, 1.2 g of chitin tetramer, 0.8 g of chitin pentamer, 0.5 g of chitin hexamer, 0.4 g of chitin heptamer or octamer and 0.3 g of chitin nonamer or decamer.

### Example 5

Forty grams of 20% hydrofluoric acid free of a surfactant was placed into a PFA container equipped with a stirrer. The air of the atmosphere was replaced by nitrogen gas. Then 10 g of chitin (Chimica Chitin F, product of Chimica Co., Ltd.) was placed into the container. After a lapse of 2 minutes, the mixture of chitin and hydrofluoric acid was uneven. Therefore, 50 g of 20% hydrofluoric acid was added. After a lapse of 2 minutes, the system became uniform, and a reaction was carried out at 80°C for 6 hours. Then 100 g of pure water was added, and undissolved components were removed by filtration. The filtrate was neutralized with 33.4 of calcium hydroxide in an ice bath. The solution had a pH of 9. After the solution was separated into solid and liquid by re-filtration, 0.1 N hydrochloric acid was added so as to attain a pH of 5. The solution was passed through a column 40 mm in diameter which was filled with 500 g of activated carbon (product of Wako Junyaku Co., Ltd.) useful for chromatography. Pure water (1000 g) was passed through the column and an aqueous solution (3000 g) of methanol with a linear concentration gradient of methanol concentration 5% to 80% was passed through the column. The solution flowing out from the column was fractionally recovered and concentrated under reduced pressure, giving 2.1 g of chitin dimer, 1.5 g of chitin trimer, 1.0 g of chitin tetramer, 0.7 g of chitin pentamer, 0.4 g of chitin hexamer, 0.4 g of chitin heptamer or octamer and 0.4 g of chitin nonamer or decamer.

### Example 6

Forty grams of 55% hydrofluoric acid without a surfactant was placed into a PFA container equipped with a stirrer. The air of the atmosphere was replaced by nitrogen gas. Then 10 g of chitin (Chimica Chitin F, product of Chimica Co., Ltd.) was placed into the container. After a lapse of 3 minutes, the chitin and hydrofluoric acid became uniform. A reaction was carried out at 35°C for 4 hours. Then 100 g of pure water was added, and undissolved components were removed by filtration. The filtrate was neutralized with 55.1 of calcium carbonate in an ice bath. The solution had a pH of 9. After the solution was separated into solid and liquid by re-filtration, 0.1 N hydrochloric acid was added so as to attain a pH of 5. The solution was passed through a column 40 mm in diameter which was filled with 500 g of activated carbon (product of Wako Junyaku Co., Ltd.) useful for chromatography. Pure water (1000 g) was passed through the column and an aqueous solution (3000 g) of ethanol with a linear concentration gradient of ethanol concentration 5% to 50% was passed through the column.
The solution flowing out from the column was fractionally recovered and concentrated under reduced pressure, giving 2.5 g of chitin dimer, 1.8 g of chitin trimer, 1.2 g of chitin tetramer, 0.6 g of chitin pentamer, 0.4 g of chitin hexamer, 0.4 g of chitin heptamer or octamer and 0.2 g of chitin nonamer or decamer.

### Example 7

Forty grams of 55% hydrofluoric acid containing 50 ppm of Leodol TW-L106 (surfactant manufactured by Kao Corp.) was placed into a PFA container equipped with a stirrer. The air of the atmosphere was replaced by nitrogen gas. Then 10 g of chitin (produced from lobsters caught in Thailand, water 8% and ash 0.5%) was placed into the container. In about 10 seconds, the chitin and hydrofluoric acid became uniform. A reaction was carried out in this state at 40°C for 3.5 hours. Then 40 g of acetone and 120 g of water were added, and undissolved components were removed by filtration. The filtrate was neutralized with 55.1 of calcium carbonate in an ice bath. The solution had a pH of 9. After the solution was separated into solid and liquid by re-filtration, 0.1 N hydrochloric acid was added so as to attain a pH of 5. The solution was passed through a medium pressure preparative liquid CROSYSTEM YFLC-8005-WD-FD (product of Yamazen Co., Ltd.) equipped with ULTRAPACK ODS-A (product of Yamazen Co., Ltd.) 50 mm in diameter and 300 mm in length, together with pure water. The solution flowing out from the column was fractionally recovered and concentrated under reduced pressure, giving 2.4 g of chitin dimer, 1.8 g of chitin trimer, 1.2 g of chitin tetramer, 0.7 g of chitin pentamer, 0.5 g of chitin hexamer, 0.4 g of chitin heptamer or octamer and 0.2 g of chitin nonamer or decamer.

### Example 8

Forty grams of 50% hydrofluoric acid containing 100 ppm of Excel O-95R (surfactant manufactured by Kao Corp.) was placed into a PFA container equipped with a stirrer. The air of the atmosphere was replaced by nitrogen gas. Then 10 g of chitin (manufactured by Hokkaido Soda Co., Ltd., North Chitin CG-2). The chitin and hydrofluoric acid became uniform in about 10 seconds. A reaction was carried out in this state at 35°C for 6 hours. Then 100 g of water was added, and undissolved components were removed by filtration. The filtrate was neutralized with 60 g of calcium hydroxide in an ice bath. The filtrate had a pH of 14 when the solution was separated into solid and liquid by re-filtration. The filtrate was left to stand at 40°C for two weeks to continue the deacetylation reaction. 0.1N hydrochloric acid was added so as to attain a pH of 5. The undissolved components were removed by filtration. The filtrate was passed through a medium pressure preparative liquid CROSYSTEM YFLC-8005-WD-FD (product of Yamazen Co., Ltd.) equipped with ULTRAPACK ODS-A (product of Yamazen Co., Ltd.) 50 mm in diameter and 300 mm in length, together with pure water. The solution flowing out from the column was fractionally recovered and concentrated under reduced pressure, giving 2.0 g of chitosan hydrochloride dimer, 1.5 g of chitosan hydrochloride trimer, 1.0 g of chitosan hydrochloride tetramer, 0.6 g of chitosan hydrochloride pentamer, 0.5 g of chitosan hydrochloride hexamer, 0.5 g of chitosan hydrochloride heptamer or octamer and 0.4 g of chitosan hydrochloride nonamer or decamer.

### Example 9

Forty grams of 50% hydrofluoric acid containing 50 ppm of Leodol TW-L106 (surfactant manufactured by Kao Corp.) was placed into a PFA container equipped with a stirrer. The air of the atmosphere was replaced by nitrogen gas. Then 10 g of chitosan (manufactured by Koyo Chemical Co., Ltd., SK-400) was placed into the container. The chitosan and hydrofluoric acid became uniform in about 10 seconds. A reaction was carried out in this state at 40°C for 5 hours. Then 100 g of pure water was added, and undissolved components were removed by filtration.
The filtrate was neutralized with 50.2 g of calcium carbonate in an ice bath. The solution had a pH of 9.
The solution was separated into solid and liquid by re-filtration. 0.1 N hydrochloric acid was added so as to attain a pH of 5. The solution was passed through a column 40 mm in diameter which was filled with 500 g of activated carbon (product of Wako Junyaku Co., Ltd.) useful for chromatography. Pure water (1000 g) was passed through the column and an aqueous solution (3000 g) of methanol with a linear concentration gradient of methanol concentration 5% to 80% was passed through the column.
The solution flowing out from the column was fractionally recovered and concentrated under reduced pressure, giving 1.8 g of chitosan hydrochloride dimer, 1.5 g of chitosan hydrochloride trimer, 1.0 g of chitosan hydrochloride tetramer, 0.6 g of chitosan hydrochloride pentamer, 0.5 g of chitosan hydrochloride hexamer, 0.5 g of chitosan hydrochloride heptamer or octamer and 0.4 g of chitosan hydrochloride nonamer or decamer.

### Comparative Example 1

250 g of 0.2% hydrofluoric acid was placed into a PFA container equipped with a stirrer. The air of the atmosphere was replaced by nitrogen gas. Then 10 g of chitin (manufactured by Hokkaido Soda Co., Ltd., North Chitin CG-2) was added. The chitin and hydrofluoric acid became uniform in about 3 minutes. A reaction was conducted at 40°C for 48 hours. Thereafter the undissolved components were removed by filtration. The filtrate was neutralized with 1.3 g of calcium carbonate in an ice bath. The solution had a pH of 9. After the solution was separated into solid and liquid by re-filtration, 0.1 N hydrochloric acid was added so as to attain a pH of 5.
The undissolved components were removed by further filtration and the filtrate was concentrated under reduced pressure, whereby 0.1 g of chitin oligomer was recovered.

### Comparative Example 2

Forty grams of hydrofluoric anhydride was placed into a PFA container equipped with a stirrer wherein the air of the atmosphere was replaced by nitrogen gas. Then 10 g of chitin (manufactured by Koyo Chemical Co., Ltd., Chitin TC-L) was placed into the container. The chitin and hydrofluoric anhydride became uniform in about 3 minutes and were reacted in this state at 0°C for three hours. Thereafter an attempt was made to add calcium carbonate for neutralization, but the temperature was abruptly elevated on addition of 1 g of calcium carbonate with ice cooling. Instantly the container became filled with hydrogen fluoride gas. The operation was stopped for danger.

### Comparative Example 3

Forty grams of hydrofluoric anhydride was placed into a PFA container equipped with a stirrer wherein the air of the atmosphere was replaced by nitrogen gas. Then 10 g of chitin (manufactured by Koyo Chemical Co., Ltd., Chitin TC-L) was placed into the container. The chitin and hydrofluoric anhydride became uniform in about 3 minutes and were reacted in this state at 0°C for three hours. Thereafter an attempt was made to dilute the mixture with ice-cooled pure water to become ready for neutralization, but the temperature was abruptly elevated on dropwise addition of pure water. Instantly the container became filled with hydrogen fluoride gas. The operation was stopped for danger.

### Comparative Example 4

Forty grams of hydrofluoric anhydride was placed into a PFA container equipped with a stirrer wherein the air of the atmosphere was replaced by nitrogen gas. Then 10 g of chitin (manufactured by Koyo Chemical Co., Ltd., Chitin TC-L) was placed into the container. The chitin and hydrofluoric anhydride became uniform in about 3 minutes and were reacted in this state at 0°C for three hours.
The mixture of chitin and hydrofluoric anhydride was added dropwise to 40 g of ice-cooled pure water to dilute the mixture so that it became ready for neutralization. Hydrogen fluoride gas was generated at the surface of the liquid every time each drop of the mixture fell upon the surface. The drop was slowly made to fall on the surface carefully. It took 1.5 hours to drop the total mixture.

A chitin oligomer and/or a chitosan oligomer can be efficiently prepared in Examples 1-7 illustrative of the invention.

It was found that in Examples 3, 4, and 7-9 using the surfactants, a higher initial wetting was exhibited between hydrofluoric acid and chitin and/or chitosan than in Examples 5 and 6 without use of a surfactant. This means that the chitin and/or chitosan non-contacted with hydrofluoric acid can be highly prevented from adhering to the side wall of the reactor or to the propeller member of the stirrer and that the distribution of polymerization degree of the chitin oligomer and/or the chitosan oligomer contacted (mixed) with hydrofluoric acid for a specified time can be effectively kept constant invariably.

Aside from the above, when the procedures of Examples 3-9 were conducted repeatedly ten times in the same manner, an oligomer of each polymerization degree was produced in a constant yield with high reproducibility.
It is noted that the oligomers prepared using the surfactants in Examples 3, 4 and 7-9 exhibited a particularly high reproducibility.

The chitin or chitosan oligomers prepared in Examples 3-9 were evaluated with ION CHROMATO DX500 (column; IONPACAS 17) manufactured by DIONEX Co., Ltd. It was found that the concentrations of fluorine remaining in the chitin oligomers obtained in Examples 3-8 or chitosan oligomers prepared by deacetylation of chitin oligomers in excess alkali component were 0.001 to 10 ppm, whereas the concentration of fluorine remaining in the chitosan oligomer obtained in Example 9 was 20 ppm, which shows that the concentration of remaining fluorine can be more efficiently reduced when using a chitin-based material. The following is noted. One g of calcium hydroxide was added to the chitosan oligomer produced in Example 9. After the mixture was filtered, the filtrate was concentrated under reduced pressure again to recover a chitin oligomer, and the concentration of remaining fluorine was reduced to 0.01 ppm.

The solutions containing the chitin oligomers or chitosan oligomers before passage through the column in Examples 3-9 were concentrated and dried. The concentrations of fluorine remaining in the chitin oligomers or chitosan oligomers were measured, and were found to be 20 ppm or less. The amount of remaining calcium ion as evaluated with ICP mass spectrograph SPQ 8000 (product of Seiko Co., Ltd.) in respect of Examples 3-7 and 9 excepting Example 8 using a great excess of basic calcium salt for deacetylation was 1 to 50 ppm before passage of the solution through the column and was 0.001 to 10 ppm thereafter.

Finally the concentration of endotoxin was evaluated with TOXINOMTER-ET-2000 (product of Wako Junyaku Kogyo Co., Ltd.) using LIMRUS ES-II Single Test Wako (product of Wako Junyaku Kogyo Co., Ltd.) as a reagent. The resultant values were lower than the detection limit in respect of Examples 3-9.

The solubility of the oligomers in 100 g of water at 20°C was evaluated, and was found to be 4 to 10 g in Examples 3-9.

The chitin oligomer was obtained in a very low yield in Comparative Example 1 which is different from the invention in that the concentration of hydrofluoric acid was low. It is difficult to safely remove hydrofluoric acid while controlling the temperature in Comparative Examples 2 and 3 which are different from the invention in that the concentration of hydrofluoric acid was high. It was found that too much time was taken until neutralization in Comparative Example 4. Presumably the polymerization degree of the contemplated oligomer is difficult to control in the method of Comparative Example 4 significantly different in the time for contact with concentrated hydrofluoric acid.

### Example 10

Forty grams of 50% hydrofluoric acid containing 25 ppm of Leodol TW-L106 (surfactant manufactured by Kao Corp.) and 10 g of 36% hydrochloric acid were placed into a PFA container equipped with a stirrer. The air of the atmosphere was replaced by nitrogen gas. Then 10 g of chitosan (manufactured by Koyo Chemical Co., Ltd., SK-200, deacetylation degree=0.8 or more) was placed into the container. The chitosan and hydrohalogenated acid became uniform in about 10 seconds. A reaction was carried out in this state at 40°C for 3 hours. Then 100 g of pure water was added, and undissolved components were removed by filtration. The filtrate was neutralized with 40.8 g of calcium hydroxide in an ice bath. The solution had a pH of 9. After the solution was separated into solid and liquid by re-filtration, 0.1 N hydrochloric acid was added so as to attain a pH of 5. The solution was passed through a medium pressure preparative liquid CROSYSTEM YFLC 8005-WD-FD (product of Yamazen Co., Ltd.) equipped with ULTRAPACK ODS-A (product of Yamazen Co., Ltd.) 50 mm in diameter and 300 mm in length, together with pure water. The solution flowing out from the column was fractionally recovered and concentrated under reduced pressure, giving 2.5 g of chitosan hydrochloride dimer, 1.8 g of chitosan hydrochloride trimer, 1.2 g of chitosan hydrochloride tetramer, 0.8 g of chitosan hydrochloride pentamer, 0.5 g of chitosan hydrochloride hexamer, 0.4 g of chitosan hydrochloride heptamer or octamer and 0.3 g of chitosan hydrochloride nonamer or decamer.

### Example 11

Ten g of chitosan (product of Koyo Chemical Co., Ltd., FM-200, deacetylation degree =0.8 or more) was placed into a PFA container equipped with a stirrer. The air of the atmosphere was replaced by nitrogen gas. Then 25 g of 40% hydrofluoric acid containing 10 ppm of Emazol L-10 (surfactant manufactured by Kao Corp.) and 25 g of 10% hydrochloric acid were placed into the container at the same time. The chitosan and hydrohalogenated acid became uniform in about 10 seconds. A reaction was carried out in this state at 70°C for 4 hours. Then 80 g of pure water was added, and undissolved components were removed by filtration. The filtrate was neutralized with 21.1 g of calcium hydroxide in an ice bath. The solution had a pH of 8. After the solution was separated into solid and liquid by re-filtration, 0.1 N hydrochloric acid was added so as to attain a pH of 5. The solution was passed through a medium pressure preparative liquid CROSYSTEM YFLC 8005-WD-FD (product of Yamazen Co., Ltd.) equipped with ULTRAPACK ODS-A (product of Yamazen Co., Ltd.) 50 mm in diameter and 300 mm in length, together with pure water. The solution flowing out from the column was fractionally recovered and concentrated under reduced pressure, giving 2.0 g of chitosan hydrochloride dimer, 1.5 g of chitosan hydrochloride trimer, 1.0 g of chitosan hydrochloride tetramer, 0.7 g of chitosan hydrochloride pentamer, 0.5 g of chitosan hydrochloride hexamer, 0.5 g of chitosan hydrochloride heptamer or octamer and 0.5 g of chitosan hydrochloride nonamer or decamer.

### Example 12

Forty grams of 50% hydrofluoric acid without a surfactant and 20 g of 36% hydrochloric acid were placed into a PFA container equipped with a stirrer. The air of the atmosphere was replaced by nitrogen gas. Then 10 g of chitosan (product of Kyowa Technos Co., Ltd., FLOWNACK H, deacetylation degree=0.8 or more) was placed into the container. Since 20 g of 36% hydrochloric acid was added in view of presence of chitosan mass non-contacted therewith even after 2 minutes. The system became uniform after 2 minutes. A reaction was performed in this state at 40°C for 2 hours. Then 100 g of pure water was added, and undissolved components were removed by filtration. The filtrate was neutralized with 51.7 g of calcium hydroxide in an ice bath. The solution had a pH of 9. After the solution was separated into solid and liquid by re-filtration, 0.1 N hydrochloric acid was added so as to attain a pH of 5. The solution was passed through a medium pressure preparative liquid CROSYSTEM YFLC-8005-WD-FD (product of Yamazen Co., Ltd.) equipped with ULTRAPACK ODS-A (product of Yamazen Co., Ltd.) 50 mm in diameter and 300 mm in length, together with pure water. The solution flowing out from the column was fractionally recovered and concentrated under reduced pressure, giving 1.8 g of chitosan hydrochloride dimer, 1.4 g of chitosan hydrochloride trimer, 1.0 g of chitosan hydrochloride tetramer, 0.9 g of chitosan hydrochloride pentamer, 0.7 g of chitosan hydrochloride hexamer, 0.5 g of chitosan hydrochloride heptamer or octamer and 0.5 g of chitosan hydrochloride nonamer or decamer.

### Example 13

One hundred grams of a mixture of 20% hydrofluoric acid without a surfactant and 15% hydrochloric acid was placed into a PFA container equipped with a stirrer. The air of the atmosphere was replaced by nitrogen gas. Then 10 g of chitosan (product of Koyo Chemical Co., Ltd., FM-200, deacetylation degree=0.8 or more) was placed into the container. After 3 minutes, the chitosan and hydrohalogenated acid became uniform, and a reaction was performed in this state at 65°C for 4 hours. Then 100 g of pure water was added, and undissolved components were removed by filtration. The filtrate was neutralized with 70.6 g of calcium carbonate in an ice bath. The solution had a pH of 9. After the solution was separated into solid and liquid by re-filtration, 0.1 N hydrochloric acid was added so as to attain a pH of 5. The solution was passed through a medium pressure preparative liquid CROSYSTEM YFLC-8005-WD-FD (product of Yamazen Co., Ltd.) equipped with ULTRAPACK ODS-A (product of Yamazen Co., Ltd.) 50 mm in diameter and 300 mm in length, together with pure water. The solution flowing out from the column was fractionally recovered and concentrated under reduced pressure, giving 1.8 g of chitosan hydrochloride dimer, 1.2 g of chitosan hydrochloride trimer, 0.8 g of chitosan hydrochloride tetramer, 0.6 g of chitosan hydrochloride pentamer, 0.4 g of chitosan hydrochloride hexamer, 0.5 g of chitosan hydrochloride heptamer or octamer and 0.4 g of chitosan hydrochloride nonamer or decamer.

### Example 14

One hundred grams of a mixture of 20% hydrofluoric acid containing 25 ppm of Leodol TW-L106 (surfactant manufactured by Kao Corp.) and 15% hydrochloric acid was placed into a PFA container equipped with a stirrer. The air of the atmosphere was replaced by nitrogen gas. Then 10 g of chitosan (product of Koyo Chemical Co., Ltd., FM-200, deacetylation degree=0.8 or more) was placed into the container. In about 10 seconds, the chitosan and hydrohalogenated acid became uniform. Thereafter, a reaction was carried out in this state at 65°C for 4 hours. Then 50 g of pure water and 50 g of methanol were added, and undissolved components were removed by filtration. The filtrate was neutralized with 70.6 g of calcium carbonate in an ice bath. The solution had a pH of 9. After the solution was separated into solid and liquid by re-filtration, 0.1 N hydrochloric acid was added so as to attain a pH of 5. The solution was passed through a medium pressure preparative liquid CROSYSTEM YFLC-8005-WD-FD (product of Yamazen Co., Ltd.) equipped with ULTRAPACK ODS-A (product of Yamazen Co., Ltd.) 50 mm in diameter and 300 mm in length, together with pure water. The solution flowing out from the column was fractionally recovered and concentrated under reduced pressure, giving 1.8 g of chitosan hydrochloride dimer, 1.2 g of chitosan hydrochloride trimer, 0.8. g of chitosan hydrochloride tetramer, 0.6 g of chitosan hydrochloride pentamer, 0.5 g of chitosan hydrochloride hexamer, 0.5 g of chitosan hydrochloride heptamer or octamer and 0.4 g of chitosan hydrochloride nonamer or decamer.

### Example 15

Forty grams of a mixture of 4% hydrofluoric acid containing 25 ppm of Leodol TW-L106 (surfactant manufactured by Kao Corp.) and 25% hydrochloric acid was placed into a PFA container equipped with a stirrer. The air of the atmosphere was replaced by nitrogen gas. Then 10 g of chitosan (product of Koyo Chemical Co., Ltd., FM-200, deacetylation degree=0.8 or more) was placed into the container. In about 10 seconds, the chitosan and hydrohalogenated acid became uniform. Thereafter a reaction was carried out in this state at 60°C for 4 hours. Then 50 g of pure water and 50 g of methanol were added, and undissolved components were removed by filtration.
The filtrate was neutralized with 17.8 g of calcium carbonate in an ice bath. The solution had a pH of 9. After the solution was separated into solid and liquid by re-filtration, 0.1 N hydrochloric acid was added so as to attain a pH of 5. The solution was passed through a medium pressure preparative liquid CROSYSTEM YFLC-8005-WD-FD (product of Yamazen Co., Ltd.) equipped with ULTRAPACK ODS-A (product of Yamazen Co., Ltd.) 50 mm in diameter and 300 mm in length, together with pure water. The solution flowing out from the column was fractionally recovered and concentrated under reduced pressure, giving 2.0 g of chitosan hydrochloride dimer, 1.4 g of chitosan hydrochloride trimer, 1.0 g of chitosan hydrochloride tetramer, 0.6 g of chitosan hydrochloride pentamer, 0.3 g of chitosan hydrochloride hexamer, 0.3 g of chitosan hydrochloride heptamer or octamer and 0.2 g of chitosan hydrochloride nonamer or decamer.

### Example 16

Forty grams of 50% hydrofluoric acid containing 25 ppm of Leodol TW-L106 (surfactant manufactured by Kao Corp.) and 10 g of 36% hydrochloric acid were placed into a PFA container equipped with a stirrer. The air of the atmosphere was replaced by nitrogen gas. Then 10 g of chitin (manufactured by Chimica, Co., Ltd., Chimica Chitin F, deacetylation degree=0.1 or less) was placed into the container. In about 10 seconds, the chitin and hydrohalogenated acid became uniform. Thereafter a reaction was carried out in this state at 40°C for 3 hours. Then 100 g of pure water was added, and undissolved components were removed by filtration. The filtrate was neutralized with 40.8 g of calcium hydroxide in an ice bath. The solution had a pH of 9. After the solution was separated into solid and liquid by re-filtration, 0.1 N hydrochloric acid was added so as to attain a pH of 5. The solution was passed through a column 40 mm in diameter which was filled with 500 g of activated carbon (product of Wako Junyaku Co., Ltd.) useful for chromatography. Pure water (1000 g) was passed through the column and an aqueous solution (3000 g) of methanol with a linear concentration gradient of methanol concentration 5% to 80% was passed through the column. The solution flowing out from the column was fractionally recovered and concentrated under reduced pressure, giving 2.2 g of chitin dimer, 1.6 g of chitin trimer, 1.1 g of chitin tetramer, 0.7 g of chitin pentamer, 0.4 g of chitin hexamer, 0.3 g of chitin heptamer or octamer and 0.3 g of chitin nonamer or decamer.

### Comparative Example 5

250 g of a mixture of 0.2% hydrofluoric acid and 0.2% hydrochloric acid was placed into a PFA container equipped with a stirrer. The air of the atmosphere was replaced by nitrogen gas. Then 10 g of chitosan (product of Koyo Chemical Co., Ltd., FM-200) was placed into the container. In about 3 minutes, the chitosan and hydrohalogenated acid became uniform. A reaction was carried out in this state at 40°C for 48 hours. Then, undissolved components were removed by filtration. The filtrate was neutralized with 2.0 g of calcium carbonate in an ice bath. The solution had a pH of 9. After the solution was separated into solid and liquid by re-filtration, 0.1 N hydrochloric acid was added so as to attain a pH of 5. The undissolved components were removed by further filtration and the filtrate was concentrated under reduced pressure, whereby 0.1 g of chitosan oligomer was recovered.

A chitin oligomer and/or a chitosan oligomer can be efficiently prepared in Examples 10-16 illustrating the invention.

It was found that in Examples 10, 11 and 14-16 using surfactants, a high initial wetting was exhibited between hydrofluoric acid and chitin and/or chitosan compared with Examples 12 and 13 carried out without using a surfactant. This shows that the chitin and/or chitosan non-contacted with hydrohalogenated acid can be highly prevented from adhering to the side wall of the reactor or to the propeller member of the stirrer and that the distribution of polymerization degree of the chitin oligomer and/or chitosan oligomer contacted (mixed) for a specified time can be effectively kept constant invariably.

Aside from the above, when the procedures of Examples 10 to 16 were conducted repeatedly ten times in the same manner, an oligomer of each polymerization degree was prepared in a constant yield with high reproducibility. It is noted that the oligomers prepared using surfactants in Examples 10, 11 and 14-16 exhibited a particularly high reproducibility.

The chitin or chitosan oligomers prepared in Examples 10-16 were evaluated with ION CHROMATO DX500 (column; IONPACAS 17) manufactured by DIONEX Co., Ltd. It was found that the concentrations of fluorine remaining in the chitin oligomers and chitosan oligomers obtained in Examples 10-16 were 0.001 to 10 ppm. According to the inventors' knowledge, a chitosan oligomer is ready to form a fluoric salt and generally displays a higher concentration of remaining fluorine than a chitin. The chitosan oligomers prepared in the invention had low concentrations of remaining fluorine component. The invention was found to achieve a remarkable effect in preparing a chitosan oligomer composition from a chitosan-based material. The concentration of remaining metal was evaluated with ICP mass spectrograph SPQ 8000 (product of Seiko Co., Ltd.). It was found that the concentration of remaining calcium in Example 15 was higher than in Examples 10-14 and 16, namely about 200 ppm (about 10 to about 50 ppm in other Examples), because the concentration of hydrochloric acid is higher than the concentration of hydrofluoric acid in Example 15. However, the chitosan oligomer prepared in Example 6 was passed through a medium pressure preparative liquid CROSYSTEM YFLC-8005-WD-FD (product of Yamazen Co., Ltd.) equipped with ULTRAPACK ODS-A (product of Yamazen Co., Ltd.) 50 mm in diameter and 300 mm in length, together with pure water. The solution flowing out from the column was fractionally recovered and concentrated under reduced pressure. The concentration of remaining calcium was 1 ppm.

Finally the concentration of endotoxin was evaluated with TOXINOMTER ET-2000 (product of Wako Junyaku Kogyo Co., Ltd.) using LIMRUS ES-II Single Test Wako (product of Wako Junyaku Kogyo Co., Ltd.) as a reagent. The resultant values were lower than the detection limit in respect of Examples 10-16. The solubility of the oligomers in 100 g of water at 20°C was evaluated, and was found to be 4 to 12 g in Examples 3-9. It was found that a chitin oligomer was prepared in a very low yield in Comparative Example 5 which is different from the invention in that the concentration of hydrofluoric acid is low.

### Example 17

Thirty grams of 80% hydrofluoric acid containing 40 ppm of Emazol L-10 (surfactant manufactured by Kao Corp.) was placed into a PFA container equipped with a stirrer. The air of the atmosphere was replaced by nitrogen gas. Then 10 g of chitin (Chimica Chitin F, product of Chimica Co., Ltd.) was placed into the container. The chitin and hydrofluoric acid became uniform in about 15 seconds. A reaction was carried out in this state at 0°C for 1.5 hours. Then 30 g of ethanol and 70 g of pure water were added, and undissolved components were removed by filtration. The filtrate was neutralized with 44.5 g of calcium hydroxide in an ice bath. The solution had a pH of 8. After the solution was separated into solid and liquid by re-filtration, 0.1 N hydrochloric acid was added so as to attain a pH of 5. The solution was passed through a column 40 mm in diameter which was filled with 500 g of activated carbon (product of Wako Junyaku Co., Ltd.) useful for chromatography. Pure water (1000 g) was passed through the column and an aqueous solution (3000 g) of ethanol with a linear concentration gradient of ethanol concentration 5% to 50% was passed through the column. The solution flowing out from the column was fractionally recovered and concentrated under reduced pressure, giving 1.2 g of chitin monomer, 1.2 g of chitin dimer, 1.3 g of chitin trimer, 1.0 g of chitin tetramer, 0.8 g of chitin pentamer, 0.6 g of chitin hexamer, 0.8 g of chitin heptamer or octamer and 0.4 g of chitin nonamer or decamer.

### Example 18

Forty grams of 80% hydrofluoric acid without a surfactant was placed into a PFA container equipped with a stirrer. The air of the atmosphere was replaced by nitrogen gas. Then 10 g of chitin (Chimica Chitin F, product of Chimica Co., Ltd.) was placed into the container. It took 30 minutes until the chitin and hydrofluoric acid became uniform. A reaction was carried out at 0°C for 4 hours. Then 80 g of pure water was added, and an extremely small amount of undissolved components was removed by filtration. The filtrate was neutralized with 59.3 of calcium hydroxide in an ice bath. The solution had a pH of 9. After the solution was separated into solid and liquid by re-filtration, 0.1 N hydrochloric acid was added so as to attain a pH of 5. The solution was passed through a column 40 mm in diameter which was filled with 500 g of activated carbon (product of Wako Junyaku Co., Ltd.) useful for chromatography. Pure water (1000 g) was passed through the column and an aqueous solution (3000 g) of methanol with a linear concentration gradient of methanol concentration 5% to 80% was passed through the column. The solution flowing out from the column was fractionally recovered and concentrated under reduced pressure, giving 2.4 g of chitin monomer, 1.8 g of chitin dimer, 1.4 g of chitin trimer, 0.9 g of chitin tetramer, 0.6 g of chitin pentamer, 0.4 g of chitin hexamer, 0.4 g of chitin heptamer or octamer and 0.1 g of chitin nonamer or decamer.

### Example 19

Thirty grams of 90% hydrofluoric acid containing 10 ppm of Leodol TW-L106 (surfactant manufactured by Kao Corp.) was placed into a PFA container equipped with a stirrer. The air of the atmosphere was replaced by nitrogen gas. Then 10 g of chitin (Chimica Chitin F, product of Chimica Co., Ltd.) was placed into the container. The chitin and hydrofluoric acid became uniform in about 20 seconds. A reaction was carried out in this state at 27°C for 4 hours. Then 500 g of ice-cooled pure water was added dropwise, and an extremely small amount of undissolved components was removed by filtration. The filtrate was neutralized with 67.6 g of calcium carbonate in an ice bath. The solution had a pH of 9. After the solution was separated into solid and liquid by re-filtration, 0.1 N hydrochloric acid was added so as to attain a pH of 5. The solution was passed through a column 40 mm in diameter which was filled with 500 g of activated carbon (product of Wako Junyaku Kogyo Co., Ltd.) useful for chromatography. Pure water (1000 g) was passed through the column and an aqueous solution (3000 g) of ethanol with a linear concentration gradient of ethanol concentration 5% to 50% was passed through the column. The solution flowing out from the column was fractionally recovered and concentrated under reduced pressure, giving 9.5 g of chitin monomer, and 0.4 g of chitin oligomer dimer or higher.

### Example 20

Forty grams of 85% hydrofluoric acid containing 50 ppm of Leodol TW-L106 (surfactant manufactured by Kao Corp.) was placed into a PFA container equipped with a stirrer. The air of the atmosphere was replaced by nitrogen gas. Then 10 g of chitin (produced from lobsters caught in Thailand, water 8% and ash 0.5%) was placed into the container. In about 10 seconds, the chitin and hydrofluoric acid became uniform. A reaction was carried out in this state at -20°C for 1 hour. Then 120 g of pure water was added, and undissolved components were removed by filtration. The filtrate was neutralized with 85.1 of calcium carbonate in an ice bath. The solution had a pH of 9. After the solution was separated into solid and liquid by re-filtration, 0.1 N hydrochloric acid was added so as to attain a pH of 5. The solution was passed through a medium pressure preparative liquid CROSYSTEM YFLC-8005-WD-FD (product of Yamazen Co., Ltd.) equipped with ULTRAPACK ODS-A (product of Yamazen Co., Ltd.) 50 mm in diameter and 300 mm in length, together with pure water. The solution flowing out from the column was fractionally recovered and concentrated under reduced pressure, giving 0.5 g of chitin monomer, 1.0 g of chitin dimer, 0.9 g of chitin trimer, 0.9 g of chitin tetramer, 0.9 g of chitin pentamer, 0.7 g of chitin hexamer, 0.8 g of chitin heptamer or octamer and 0.4 g of chitin nonamer or decamer.

### Example 21

Forty grams of 75% hydrofluoric acid containing 100 ppm of Excel O-95R (manufactured by Kao Corp.) was placed into a PFA container equipped with a stirrer. The air of the atmosphere was replaced by nitrogen gas. Then 10 g of chitin (manufactured by Hokkaido Soda Co., Ltd., North Chitin CG-2) was placed into the container. The chitin and hydrofluoric acid became uniform in about 10 seconds. A reaction was carried out in this state at 0°C for 2 hours. Then 100 g of pure water was added, and undissolved components were removed by filtration. The filtrate was neutralized with 65 g of calcium hydroxide in an ice bath. The filtrate had a pH of 14 when the solution was separated into solid and liquid by re-filtration. The filtrate was left to stand at 40°C for two weeks to continue the deacetylation reaction. 0.1N hydrochloric acid was added so as to attain a pH of 5. The undissolved components were removed by filtration. The filtrate was passed through a medium pressure preparative liquid CROSYSTEM YFLC-8005-WD-FD (product of Yamazen Co., Ltd.) equipped with ULTRAPACK ODS-A (product of Yamazen Co., Ltd.) 50 mm in diameter and 300 mm in length, together with pure water. The solution flowing out from the column was fractionally recovered and concentrated under reduced pressure, giving 1.2 g of chitosan hydrochloride monomer, 1.3 g of chitosan hydrochloride dimer, 1.3 g of chitosan hydrochloride trimer, 1.1 g of chitosan hydrochloride tetramer, 0.8 g of chitosan hydrochloride pentamer, 0.6 g of chitosan hydrochloride hexamer, 0.8 g of chitosan hydrochloride heptamer or octamer and 0.5 g of chitosan hydrochloride nonamer or decamer.

### Example 22

Forty grams of 80% hydrofluoric acid containing 50 ppm of Leodol TW-L106 (surfactant manufactured by Kao Corp.) was placed into a PFA container equipped with a stirrer. The air of the atmosphere was replaced by nitrogen gas. Then 10 g of chitosan (manufactured by Koyo Chemical Co., Ltd., SK-400) was placed into the container. The chitosan and hydrofluoric acid became uniform in about 10 seconds. A reaction was carried out in this state at 20°C for 4 hours. Then 100 g of pure water was added, and undissolved components were removed by filtration. The filtrate was neutralized with 80.1 g of calcium carbonate in an ice bath. The solution had a pH of 9. After the solution was separated into solid and liquid by re-filtration, 0.1N hydrochloric acid was added so as to attain a pH of 5. The solution was passed through a medium pressure preparative liquid CROSYSTEM YFLC-8005-WD-FD (product of Yamazen Co., Ltd.) equipped with ULTRAPACK ODS-A (product of Yamazen Co., Ltd.) 50 mm in diameter and 300 mm in length, together with pure water. The solution flowing out from the column was fractionally recovered and concentrated under reduced pressure, giving 1.7 g of chitosan hydrochloride monomer, 1.6 g of chitosan hydrochloride dimer, 1.6 g of chitosan hydrochloride trimer, 1.2 g of chitosan hydrochloride tetramer, 0.9 g of chitosan hydrochloride pentamer, 0.8 g of chitosan hydrochloride hexamer, 1.1 g of chitosan hydrochloride heptamer or octamer and 0.6 g of chitosan hydrochloride nonamer or decamer.

### Comparative Example 6

Forty grams of hydrofluoric anhydride was placed into a PFA container equipped with a stirrer wherein the air of the atmosphere was replaced by nitrogen gas. Then 10 g of chitin (manufactured by Koyo Chemical Co., Ltd., Chitin TC-L) was added. The chitin and hydrofluoric anhydride became uniform in about 3 minutes and were reacted in this state at 0°C for three hours. Thereafter an attempt was made to add calcium carbonate for neutralization, but the temperature was abruptly elevated on addition of 1 g of calcium carbonate with ice cooling. Instantly the container became filled with hydrogen fluoride gas. The operation was stopped for danger.

### Comparative Example 7

Forty grams of hydrofluoric anhydride was placed into a PFA container equipped with a stirrer wherein the air of the atmosphere was replaced by nitrogen gas. Then 10 g of chitin (manufactured by Koyo Chemical Co., Ltd., Chitin TC-L) was placed into the container. The chitin and hydrofluoric anhydride became uniform in about 3 minutes. A reaction was performed in this state at 0°C for three hours. Thereafter an attempt was made to dilute the mixture with ice-cooled pure water to become ready for neutralization. But the temperature was abruptly elevated on dropwise addition of pure water. Instantly the container became filled with hydrogen fluoride gas. The operation was stopped for danger.

### Comparative Example 8

Forty grams of hydrofluoric anhydride was placed into a PFA container equipped with a stirrer wherein the air of the atmosphere was replaced by nitrogen gas. Then 10 g of chitin (manufactured by Koyo Chemical Co., Ltd., Chitin TC-L) was placed into the container. The chitin and hydrofluoric anhydride became uniform in about 3 minutes. A reaction was carried out in this state at 0°C for three hours. The mixture of chitin and hydrofluoric anhydride was added dropwise to 40 g of ice-cooled pure water for dilution to become ready for neutralization. Hydrogen fluoride gas was generated at the liquid surface every time each drop of the mixture fell upon the surface. The mixture was slowly dropped onto the surface carefully. In 1.5 hours, the total mixture was dropped.

A chitin oligomer and/or a chitosan oligomer can be efficiently prepared in Examples 17-22 illustrating the invention.

It was found that in Examples 17, and 19-22 using each surfactant, a high initial wetting was exhibited between hydrofluoric acid and chitin and/or chitosan compared with Example 18 not using a surfactant. This shows that the chitin and/or chitosan non-contacted with hydrofluoric acid can be highly prevented from adhering to the side wall of the reactor or to the propeller member of the stirrer and that the distribution of polymerization degree of the chitin oligomer and/or chitosan oligomer contacted (mixed) with hydrofluoric acid for a specified time can be effectively kept constant invariably.

Aside from the above, when in respect of Examples 17-22, the procedures were conducted repeatedly ten times in the same manner, an oligomer of each polymerization degree was produced in a constant yield with high reproducibility. It is also noted that the oligomers containing surfactants in Examples 17 and 19-22 showed a particularly high reproducibility. The present inventors' experiments confirmed that it is difficult to achieve such reproducibility by hydrolysis of a chitin-based material and/or a chitosan-based material using an enzyme. Such reproducibility can not be easily attained by hydrolysis of a chitin-based material and/or a chitosan-based material using hydrochloric acid alone.

The chitin oligomers or chitosan oligomers prepared in Examples 17-22 were evaluated with ION CHROMATO DX500 (column; IONPACAS 17) manufactured by DIONEX Co., Ltd. It was found that the concentrations of fluorine remaining in the chitin oligomers obtained in Examples 17-21 or chitosan oligomers prepared by deacetylation of chitin oligomers in an excess alkali component, respectively were 0.001 to 10 ppm, whereas the concentration of fluorine remaining in the chitosan oligomer of Example 22 was 20 ppm, which shows that the concentration of remaining fluorine can be more efficiently reduced when using a chitin-based material as the raw material. The following is noted. After 1 g of calcium hydroxide was added to the chitosan oligomer produced in Example 22, the mixture was filtered and the filtrate was concentrated under reduced pressure again to recover a chitin oligomer. Then the concentration of remaining fluorine was reduced to 0.1 ppm.

The solution containing the chitin oligomer or chitosan oligomer before passage through the column in Examples 17-21 was concentrated and dried. The concentration of fluorine remaining in the chitin oligomer or chitosan oligomer was measured, and was found to be 20 ppm or less. The amount of remaining calcium ion as evaluated with ICP mass spectrograph SPQ 8000 (product of Seiko Co., Ltd.) in respect of Examples 17-20 and 22 excepting Example 21 using a great excess of basic calcium salt for deacetylation was 45 ppm before passage through the column and was 0.01 ppm after passage.

Finally the concentration of endotoxin was evaluated with TOXINOMETER-ET-2000 (manufactured by Wako Junyaku Kogyo Co., Ltd.) using LIMRUS ES-II Single Test Wako (manufactured by Wako Junyaku Kogyo Co., Ltd.) as a reagent. The resultant values were lower than the detection limit in respect of Examples 17-23. The solubility of the oligomers in 100 g of water at 20°C was evaluated, and was found to be 4 to 12 g in Examples 17-22.

It is difficult to safely reduce the concentration of hydrofluoric acid while controlling the temperature in Comparative Examples 6 and 7 which are different from the invention in that the concentration of hydrofluoric acid was higher than in the invention. It was found that too much time was taken until neutralization in Comparative Example 8. Presumably the polymerization degree of the contemplated oligomer is difficult to control in the method of Comparative Example 8 wherein the oligomer became irregular in the distribution of polymerization degree while in contact with concentrated hydrofluoric acid.

### Example 23

The chitosan monomer (glucosamine) hydrochloride and chitosan oligomer hydrochloride obtained in Example 22 were dissolved in 500 g of water. Then a concentrated solution of sodium ascorbate was added until cease of precipitation. After the mixture was filtered, the filtrate was heated under reduced pressure. Feeds for fishes, feeds for poultry, feeds for domestic animals or the like containing 0.05 to 1% of the obtained chitosan monomer (glucosamine) ascorbate and chitosan oligomer ascorbate respectively, contributed to healthy growth of individual animals. Especially the feeds were able to markedly reduce (to 10% or less) the mortality of raised fishes (e.g., red sea bream and yellowtail).

### Example 24

The chitosan monomer (glucosamine) hydrochloride and chitosan oligomer hydrochloride prepared in Example 22 were dissolved in 500 g of water. A concentrated solution of sodium lactate was added until cease of precipitation. After filtration, the filtrate was heated under reduced pressure. Feeds for fishes, feeds for poultry, feeds for domestic animals or the like containing 0.05 to 1% of the obtained chitosan monomer (glucosamine) lactate and chitosan oligomer lactate, respectively contributed to healthy growth of individual animals. Especially the feeds were able to markedly reduce the mortality of fishes bred in a large-scale fish farm (e.g., red sea bream and yellowtail) and invariably a yield of 90% or higher was achieved.

### Example 25

One % by weight of the chitosan monomer (glucosamine) hydrochloride and chitosan oligomer hydrochloride prepared in Example 22 was dissolved in a mixture of amino acids prepared by retaining 150 g of glycine, 147 g of L-glutamic acid and 500 g of water at 100°C for 100 hours. The solution is a combination of a seasoning and a nutrient having a sweet taste like vinegar-sea tangle and possessing a colorless to pale yellow color. Marine animals and insects have a marked liking for them. The solution increased the feed efficiency and reduced the mortality to less than 1/3 the current level.

### Example 26

Two % by weight of the chitosan monomer (glucosamine) hydrochloride and chitosan oligomer hydrochloride prepared in Example 22 (2 wt%) was dissolved in a mixture of amino acids prepared by retaining 75 g of glycine, 89 g of α-alanine, 147 g of L-glutamic acid and 500 g of water at 100°C for 100 hours. The solution was suitable as an additive for a mixed feed mainly composed of soybean oil cake.

As described above, the chitin oligomer composition and/or the chitosan oligomer composition prepared according to the invention are useful as an additive for feeds for raising animals.

### Example 27

Forty grams of 50% hydrofluoric acid containing 50 ppm of Leodol TW-L106 (surfactant manufactured by Kao Corp.) was placed into a PFA container equipped with a stirrer. The air of the atmosphere was replaced by nitrogen gas. Then 10 g of a mixture of chitin oligomers (polymerization degree 10 to 20) was placed into the container. The chitin oligomer and hydrofluoric acid became uniform in about 5 seconds, and were reacted in this state at 35°C for 1 hour. Then 100 g of methanol was added and undissolved components were removed by filtration. The filtrate was neutralized with 37.1 g of calcium hydroxide in an ice bath. The solution had a pH of 9. After the solution was separated into solid and liquid by re-filtration, 0.1 N hydrochloric acid was added so as to attain a pH of 5. The solution was passed through a column 40 mm in diameter which was filled with 500 g of activated carbon (product of Wako Junyaku Kogyo Co., Ltd.) useful for chromatography. Pure water (1000 g) was passed through the column and an aqueous solution (3000 g) of methanol with a linear concentration gradient of methanol concentration 5% to 80% was passed through the column after which the solution flowing out from the column was fractionally recovered and concentrated under reduced pressure, giving 2.0 g of chitin dimer, 1.5 g of chitin trimer, 1.0 g of chitin tetramer, 0.6 g of chitin pentamer, 0.4 g of chitin hexamer, 0.4 g of chitin heptamer or octamer and 0.3 g of chitin nonamer or decamer.

### Example 28

Ten grams of a chitin oligomer (polymerization degree 10 to 20) was placed into a PFA container equipped with a stirrer. The air of the atmosphere was replaced by nitrogen gas. 25 g of 40% hydrofluoric acid containing 10 ppm of Emazol L-10 (surfactant manufactured by Kao Corp.) and 25 g of 10% hydrochloric acid were added at the same time. The chitin oligomer and hydrohalogenated acid became uniform in about 10 seconds, and a reaction was carried out in this state at 50°C for 2 hours. Then 80 g of pure water was added, and undissolved components were removed by filtration. The filtrate was neutralized with 21.1 g of calcium hydroxide in an ice bath. The solution had a pH of 8. After the solution was separated into solid and liquid by re-filtration, 0.1 N hydrochloric acid was added so as to attain a pH of 5. The solution was passed through a medium pressure preparative liquid CROSYSTEM YFLC-8005-WD-FD (product of Yamazen Co., Ltd.) equipped with ULTRAPACK ODS-A (product of Yamazen Co., Ltd.) 50 mm in diameter and 300 mm in length, together with pure water. The solution flowing out from the column was fractionally recovered and concentrated under reduced pressure, giving 2.0 g of chitin dimer, 1.5 g of chitin trimer, 1.0 g of chitin tetramer, 0.7 g of chitin pentamer, 0.5 g of chitin hexamer, 0.3 g of chitin heptamer or octamer and 0.2 g of chitin nonamer or decamer.

### Example 29

Ninety grams of 20% hydrofluoric acid without a surfactant was placed into a PFA container equipped with a stirrer. The air of the atmosphere was replaced by nitrogen gas. Then 10 g of a chitin oligmer (polymerization degree of 10 to 20) was placed into the container. After a lapse of about 1 minute, the chitin oligomer and hydrofluoric acid became uniform. A reaction was conducted at 70°C for 2 hours. Then 100 g of pure water was added, and undissolved components were removed by filtration. The filtrate was neutralized with 33.4 of calcium hydroxide in an ice bath. The solution had a pH of 9. After the solution was separated into solid and liquid by re-filtration, 0.1 N hydrochloric acid was added so as to attain a pH of 5. The solution was passed through a column 40 mm in diameter which was filled with 500 g of activated carbon (product of Wako Junyaku Kogyo Co., Ltd.) useful for chromatography. Pure water (1000 g) was passed through the column. Then an aqueous solution (3000 g) of methanol with a linear concentration gradient of methanol concentration 5% to 80% was passed through the column. The solution flowing out from the column was fractionally recovered and concentrated under reduced pressure, giving 2.1 g of chitin dimer, 1.5 g of chitin trimer, 1.0 g of chitin tetramer, 0.7 g of chitin pentamer, 0.4 g of chitin hexamer, 0.2 g of chitin heptamer or octamer and 0.1 g of chitin nonamer or decamer.

### Example 30

One hundred grams of a mixture of 20% hydrofluoric acid and 15% hydrochloric acid without a surfactant was placed into a PFA container equipped with a stirrer. The air of the atmosphere was replaced by nitrogen gas. Then 10 g of chitosan oligomer (polymerization degree 10 to 20, hydrochloride type) was placed into the container. After a lapse of 3 minutes, the chitosan oligomer and hydrohalogenated acid became uniform. A reaction was conducted in this state at 35°C for 2 hours. Then 100 g of pure water was added, and undissolved components were removed by filtration. The filtrate was neutralized with 70.6 g of calcium carbonate in an ice bath. The solution had a pH of 9. After the solution was separated into solid and liquid by re-filtration, 0.1 N hydrochloric acid was added so as to attain a pH of 5. The solution was passed through a medium pressure preparative liquid CROSYSTEM YFLC-8005-WD-FD (product of Yamazen Co., Ltd.) equipped with ULTRAPACK ODS-A (product of Yamazen Co., Ltd.) 50 mm in diameter and 300 mm in length, together with pure water. The solution flowing out from the column was fractionally recovered and concentrated under reduced pressure, giving 1.8 g of chitosan hydrochloride dimer, 1.2 g of chitosan hydrochloride trimer, 0.8 g of chitosan hydrochloride tetramer, 0.6 g of chitosan hydrochloride pentamer, 0.4 g of chitosan hydrochloride hexamer, 0.2 g of chitosan hydrochloride heptamer or octamer and 0.1 g of chitosan hydrochloride nonamer or decamer.

### Example 31

Four grams of chitobiose hydrofluoric acid salt, 3 g of chitotriose hydrofluoric acid salt, 2 g of chitotetraose hydrofluoric acid salt and 1 g of chitopentaose hydrofluoric acid salt were placed into a PFA container equipped with a stirrer. The air of the atmosphere was replaced by nitrogen gas. Then 40 g of 55% hydrofluoric acid containing 50 ppm of Leodol TW-L106 (surfactant manufactured by Kao Corp.) was placed into the PFA container. The chitosan oligomer and hydrofluoric acid quickly became uniform. A reaction was carried out in this state at 35°C for 2 hours. Then 200 g of water was added, and undissolved components were removed by filtration. The filtrate was neutralized with 55.1 g of calcium carbonate in an ice bath. The solution had a pH of 9. After the solution was separated into solid and liquid by re-filtration, 0.1 N hydrochloric acid was added so as to attain a pH of 5. The solution was passed through a medium pressure preparative liquid CROSYSTEM YFLC-8005-WD-FD (product of Yamazen Co., Ltd.) equipped with ULTRAPACK ODS-A (product of Yamazen Co., Ltd.) 50 mm in diameter and 300 mm in length, together with pure water. The solution flowing out from the column was fractionally recovered and concentrated under reduced pressure, giving 9.8 g of glucosamine hydrochloride.

### Example 32

Forty grams of 50% hydrofluoric acid containing 100 ppm of Excel O-95R (surfactant manufactured by Kao Corp.) was placed into a PFA container equipped with a stirrer. Then the air of the atmosphere was replaced by nitrogen gas. A mixture of chitin oligomers comprising 4 g of N-acetyl chitobiose, 3 g of N-acetyl chitotriose, 2 g of N-acetyl chitotetraose and 1 g of N-acetyl chitopentaose was placed into the container. The chitin oligomer and hydrofluoric acid became uniform in about 10 seconds, and a reaction was carried out in this state at 40°C for 4 hours. Then 100 g of water was added, and undissolved components were removed by filtration. The filtrate was neutralized with 50.2 g of calcium carbonate in an ice bath. The solution had a pH of 9. After the solution was separated into solid and liquid by re-filtration, 0.1N hydrochloric acid was added so as to attain a pH of 5. The solution was passed through a medium pressure preparative liquid CROSYSTEM YFLC-8005-WD-FD (product of Yamazen Co., Ltd.) equipped with ULTRAPACK ODS-A (product of Yamazen Co., Ltd.) 50 mm in diameter and 300 mm in length, together with pure water. The solution flowing out from the column was fractionally recovered and concentrated under reduced pressure, whereby 9.6 g of N-acetyl glucosamine was recovered.

### Example 33

9.6 g of N-acetyl glucosamine prepared in Example 32 was dissolved in 90 g of an aqueous solution of 10% sodium hydroxide, and was left to stand at room temperature for 2 weeks. The solution was adjusted to a pH of 6 with 1N-hydrochloric acid, desalted and concentrated under reduced pressure, whereby 9.3 g of glucosamine hydrochloride was recovered.

### Example 34

Forty grams of 50% hydrofluoric acid containing 50 ppm of Leodol TW-L106 (manufactured by Kao Corp.) was placed into a PFA container equipped with a stirrer. The air of the atmosphere was replaced by nitrogen gas. Then 2 g of a mixture of chitin oligomers (polymerization degree of 10-20), and 8 g of high molecular weight chitin (manufactured by Koyo Chemical Co., Ltd., Chitin TC-L) were placed into the container at the same time. The chitin oligomer and hydrofluoric acid became uniform in about 5 seconds, and were reacted in this state at 40°C for 2 hours. Then 100 g of methanol was added, and undissolved components were removed by filtration. The filtrate was neutralized with 37.1 g of calcium hydroxide in an ice bath. The solution had a pH of 9. After the solution was separated into solid and liquid by re-filtration, 0.1 N hydrochloric acid was added so as to attain a pH of 5. The solution was passed through a column 40 mm in diameter which was filled with 500 g of activated carbon (product of Wako Junyaku Kogyo Co., Ltd.) useful for chromatography. Pure water (1000 g) was passed through the column and an aqueous solution (3000 g) of methanol with a linear concentration gradient of methanol concentration 5% to 80% was passed through the column. The solution flowing out from the column was fractionally recovered and concentrated under reduced pressure, giving 2.1 g of chitin dimer, 1.6 g of chitin trimer, 1.2 g of chitin tetramer, 0.8 g of chitin pentamer, 0.5 g of chitin hexamer, 0.4 g of heptamer or octamer and 0.2 g of chitin nonamer or decamer.

In Examples 27-34 according to the invention, a chitin oligomer and/or a chitosan oligomer can be efficiently produced.

It was found that in Examples 27, 28, 31, 32 and 34 using surfactants, a high initial wetting was exhibited between hydrohalogenated acid and chitin and/or chitosan compared with Examples 29 and 30 not using a surfactant. This shows that the chitin and/or chitosan non-contacted with hydrohalogenated acid can be highly prevented from adhering to the side wall of the reactor or to the propeller member of the stirrer and that the distribution of polymerization degree of the chitin oligomer and/or chitosan oligomer contacted (mixed) with hydrohalogenated acid for a specified time can be effectively kept constant invariably.

When the procedures of Examples 27-32 and 34 were conducted repeatedly ten times in the same manner, an oligomer of each polymerization degree was prepared in a constant yield with high reproducibility. It is also noted that in Examples 27, 28, 31, 32, and 34 using surfactants, a particularly high reproducibility was displayed.

The chitin oligomers prepared in Examples 27-29, 32 and 34 were evaluated with ION CHROMATO DX500 (column; IONPACAS 17) manufactured by DIONEX Co., Ltd. It was found that the concentrations of fluorine remaining in the chitin oligomers obtained therein were 0.001 to 10 ppm, whereas the concentrations of fluorine remaining in the chitosan oligomers obtained in Examples 30 and 31 were 20 to 25 ppm, which shows that the concentration of remaining fluorine can be more efficiently reduced when using chitin oligomers. One g of calcium hydroxide was added to the chitosan oligomers produced in Examples 30 and 31. Then the mixture was filtered and the filtrate was concentrated under reduced pressure again to recover a chitin oligomer. It was found that the concentration of remaining fluorine was reduced to 0.02 ppm.

When the solutions containing the chitin oligomer before passage through the column in Examples 27-29, 32 and 34 were concentrated and dried, the concentrations of calcium remaining
in the obtained chitin oligomer as evaluated with ICP mass spectrograph SPQ 8000 (product of Seiko Co., Ltd.) were 0.001-10 ppm, whereas the concentrations of remaining calcium as evaluated in the same manner after passage through the column were 0.01 ppm.

On the other hand, the concentrations of calcium remaining in the chitosan oligomers prepared in Examples 30 and 31 were 20 to 30 ppm.

Finally the concentration of endotoxin was evaluated with TOXINOMETER-ET-2000 (manufactured by Wako Junyaku Kogyo Co., Ltd.) using LIMRUS ES-II Single Test Wako (manufactured Wako Junyaku Kogyo Co., Ltd.) as a reagent. The resultant vales were lower than the detection limit in respect of Examples 27-34. The solubility of the oligomers in 100 g of water at 20°C was evaluated, and was found to be 4 to 12 g in Examples 27-30 and 34.

## Claims

1. A chitin oligomer composition and/or a chitosan oligomer composition **characterized by** containing 0.001 to 1000 ppm of fluorine atom.

2. A chitin oligomer composition and/or a chitosan oligomer composition **characterized by** containing 0.001 to 1000 ppm of calcium atom.

3. The chitin oligomer composition and/or the chitosan oligomer composition according to claim 1, wherein the content of the oligomer from a trimer to a decamer is 60% or more.

4. The chitin oligomer composition and/or the chitosan oligomer composition according to any of claims 1 and 2, wherein the content of the oligomer from a tetramer to a decamer is 40% or more.

5. The chitin oligomer composition and/or the chitosan oligomer composition according to any one of claims 1 to 4, wherein the amount of the composition dissolved in 100 g of water at 20°C is 2 to 20 g.

6. The chitin oligomer composition according to any one of claims 1 to 5, wherein the concentration of endotoxin is 100 EU/ml or less.

7. The chitin oligomer composition according to any one of claims 1 to 6, wherein the chitin oligomer and/or the chitosan oligomer is a chitin oligomer composition.

8. A process for preparing a chitin oligomer composition and/or a chitosan oligomer composition, the process comprising the step of adding hydrofluoric acid in a concentration of 20 to 95 wt% to a chitin-based material and/or a chitosan-based material.

9. The process according to claim 8, wherein the concentration of hydrofluoric acid is 20 to 75 wt%.

10. The process according to claim 8, wherein the concentration of hydrofluoric acid is 75 to 95 wt%.

11. A process for preparing a chitin oligomer composition and/or a chitosan oligomer composition, the process comprising the step of adding at least one of (A) hydrofluoric acid and (B) hydrohalogenated acid other than hydrofluoric acid, the process being **characterized in that** (C) the concentration of hydrofluoric acid is 1 to 75 wt% based on the total amount of hydrohalogenated acid other than hydrofluoric acid and hydrofluoric acid; and that (D) the concentration of hydrohalogenated acid other than hydrofluoric acid is 1 to 35 wt% based on the total amount of hydrohalogenated acid other than hydrofluoric acid and hydrofluoric acid.

12. The process according to any one of claims 8 to 11, wherein the liquid temperature in adding hydrofluoric acid or hydrohalogenated acid and hydrofluoric acid to the chitin-based material and/or the chitosan-based material is adjusted to -2 to 150°C.

13. The process according to any one of claims 8 to 12, wherein basic calcium salt is added, at a suitable time, to a solution containing the chitin-based material and/or the chitosan-based material, hydrofluoric acid or hydrohalogenated acid other than hydrofluoric acid and hydrofluoric acid.

14. The process according to any one of claims 8 to 13 which includes a step of adding a hydrophilic organic solvent, at a suitable time, in an amount of 1 to 400 wt parts per 100 wt parts of hydrofluoric acid to a solution containing the chitin-based material and/or the chitosan-based material, hydrofluoric acid or hydrohalogenated acid other than hydrofluoric acid and hydrofluoric acid to remove undissolved components from the solution and a step of removing the fluorine component from the solution subjected to the foregoing step.

15. The process according to any one of claims 8 to 14 which includes a step of making basic the solution containing the chitin-based material and/or the chitosan-based material, hydrofluoric acid or hydrohalogenated acid other than hydrofluoric acid and hydrofluoric acid at a suitable time to remove undissolved components from the solution after which the solution subjected to the foregoing step is made neutral or acidic.

16. The process according to any one of claims 8 to 15, wherein in a step after removing the fluorine component from the solution containing the chitin-based material and/or the chitosan-based material, hydrofluoric acid or hydrohalogenated acid other than hydrofluoric acid and hydrofluoric acid, the solution containing the chitin oligomer and/or the chitosan oligomer is brought into contact with at least one species selected from activated carbon, alumina, silica gel, alkyl-containing silica gel, amino-containing silica gel, hydroxyl-containing silica gel, and cyano-containing silica gel.

17. The process according to any one of claims 8 to 16 which includes a step of adding a hydrophilic organic solvent in an amount of 1 to 400 wt parts per 100 wt parts of hydrofluoric acid to the solution containing the chitin-based material and/or the chitosan-based material, hydrofluoric acid or hydrohalogenated acid other than hydrofluoric acid and hydrofluoric acid to remove undissolved components from the solution and wherein after removing the fluorine component from the hydrophilic organic solvent-containing solution subjected to the foregoing step, the hydrophilic organic solvent-containing solution is brought into contact with at least one species selected from activated carbon, alumina, silica gel, alkyl-containing silica gel, amino-containing silica gel, hydroxyl-containing silica gel, and cyano-containing silica gel.

18. The process according to any one of claims 8 to 17, wherein 0.0001 to 1 wt% of a surfactant is added to the solution containing the chitin-based material and/or the chitosan-based material, hydrofluoric acid or hydrohalogenated acid other than hydrofluoric acid and hydrofluoric acid at an initial stage of contact between the chitin-based material and/or the chitosan-based material and hydrofluoric acid or hydrohalogenated acid other than hydrofluoric acid and hydrofluoric acid.

19. A process for preparing a chitin oligomer composition and/or a chitosan oligomer composition, the process being **characterized by** adding hydrohalogenated acid (other than hydrofluoric acid) to a chitin oligomer and/or a chitosan oligomer which is one of the chitin-based material and/or the chitosan-based material.

20. The process according to claim 19, wherein the chitin oligomer and/or the chitosan oligomer contains at least 70 wt% of oligomer with a polymerization degree of 2 to 40.

21. The process according to any one of claims 19 and 20, wherein the concentration of hydrohalogenated acid (other than hydrofluoric acid) is 1 to 70 wt%.

22. The process according to any one of claims 19 and 21, wherein the liquid temperature in adding hydrofluoric acid or hydrohalogenated acid (other than hydrofluoric acid) to the chitin-based material and/or the chitosan-based material is adjusted to 25 to 150°C.

23. The process according to any one of claims 19 and 22, wherein a basic calcium salt is added to the solution containing the chitin-based material and/or the chitosan-based material, and hydrohalogenated acid other than hydrofluoric acid at a suitable time.

24. The process according to any one of claims 19 to 23, which includes a step of adding a hydrophilic organic solvent, at a suitable time, in an amount of 1 to 400 wt parts per 100 wt parts of hydrofluoric acid to the solution containing the chitin-based material and/or the chitosan-based material and hydrohalogenated acid (other than hydrofluoric acid) and a step of removing the fluorine component from the solution subjected to the fregoing step.

25. The process according to any one of claims 19 to 24 which includes a step of making basic the solution containing the chitin-based material and/or the chitosan-based material, and hydrohalogenated acid other than hydrofluoric acid at a suitable time to remove undissolved components from the solution after which the solution subjected to the foregoing step is made neutral or acidic.

26. The process according to any one of claims 19 to 25, wherein in a step after removing the fluorine component from the solution containing the chitin-based material and/or the chitosan-based material and hydrohalogenated acid, the solution containing the chitin oligomer and/or the chitosan oligomer is brought into contact with at least one species selected from activated carbon, alumina, silica gel, alkyl-containing silica gel, amino-containing silica gel, hydroxyl-containing silica gel, and cyano-containing silica gel.

27. The process according to any one of claims 19 to 26 which includes a step of adding a hydrophilic organic solvent in an amount of 1 to 400 wt parts per 100 wt parts of hydrofluoric acid to the solution containing the chitin-based material and/or the chitosan-based material and hydrohalogenated acid to remove undissolved components from the solution and wherein after removing the fluorine component from the solution containing the hydrophilic organic solvent subjected to the foregoing step, the hydrophilic organic solvent-containing solution is brought into contact with at least one species selected from activated carbon, alumina, silica gel, alkyl-containing silica gel, amino-containing silica gel, hydroxyl-containing silica gel, and cyano-containing silica gel.

28. The process according to any one of claims 19 to 27, wherein 0.0001 to 1 wt% of a surfactant is added to the solution containing the chitin-based material and/or the chitosan-based material and hydrohalogenated acid other than hydrofluoric acid at an initial stage of contact between the chitin-based material and/or chitosan-based material and hydrofluoric acid.

29. A feed for marine animals mainly composed of the chitin oligomer composition and/or the chitosan oligomer composition as defined in any one of claims 1 to 6.
